# EUROPEAN PATENT APPLICATION

(11) **EP 1 816 215 A1**
(43) Date of publication of application: **08.08.2007**
(21) Application number: 06075210.2
(22) Date of filing: 01.02.2006
(51) Int. Cl.: C12Q 1/68

(54) **Disease specific ASO-probes for the detection of alpha- and beta-thalassemia mutations**

(71) Applicant: Academisch Ziekenhuis Leiden, 2333 ZA Leiden (NL)
(72) Inventor: van Moorsel, Cornelia Hillegonda Maria, 3984 JB Odijk (NL); Harteveld, Cornelis Leonard, 2402 MH Alphen aan den Rijn (NL); den Dunnen, Johannes Theodorus, 3069 LA Rotterdam (NL); Giordano, Piero Carlo, 2324 CP Leiden (NL)
(74) Representative: Winckels, Johannes Hubertus F.

(57) **Abstract**

Oligo-probes were designed for the detection of in total 66 beta- and 42 alpha-thalassemia mutations. Oligo-probes were designed as 20- or 21-mers for which the SNP-position was located in the middle of the probe (position 0). Subsequent probes were defined shifted respectively 1 to 5 nucleotides to the left or to the right, both sense and anti-sense of wild type and mutant sequence. The total number of sense and anti-sense probes for the 66 beta-globin gene specific SNP's are 2184 and for the 42 alpha-globin gene specific SNP's 1484 probes. From these the best performing wild type and mutant probe pairs were selected. The set of oligonucleotide probes for Allele Specific Oligo hybridization (ASO-probes) described, can be employed for large scale high throughput post- and neonatal screening programs aiming at prevention of these severe recessive disorders.

## Description

The invention relates to the field of hereditary disorders, more in particular to hemoglobin disorders, and the diagnosis of said disorders. More specifically, the present invention relates to the detection of mutations in the α- and β-globin genes causing α- and β-thalassemia respectively.

Thalassemia is a blood related disorder or hemoglobinopathy in which the amount of functional haemoglobin is decreased. This leads to a wide array of clinical symptoms ranging from a light form of anemia to severe forms of anemia and edema. The severity of the clinical symptoms is dependent on the mutations in the α- and β-globin genes, coding for haemoglobin. The haematological analysis typically needs verification by molecular analysis and characterization of the genotype in order to confirm the diagnosis in carriers and to offer prenatal diagnosis to couples at risk for these disorders.

The set of oligonucleotide probes for Allele Specific Oligo hybridization (ASO-probes) described in the present patent application, can be employed for large scale high throughput post- and neonatal screening programs aiming at prevention of these severe recessive disorders.

Geographical areas have developed different mutation spectra causing thalassemia. Therefore, the origin of the patient is usually included in the selection of oligo-probes to be used for the diagnosis. In the present invention, a comprehensive test is presented wherein a representative number of mutations of each area is testable. This is done by providing a series of probes that is capable of diagnosing specific thalassemia-related mutations in the alpha globin and beta-globin genes.

Selections of oligo-probes of the present invention, covers a representative number of mutations that have been found throughout the world. Such selections are, among others, suited for worldwide screening or for immigration countries harbouring a diverse population from geographic areas where thalassemia reaches high frequencies.

The hemoglobinopathies represent a large group of recessively inherited diseases, which affect the structure or synthesis of the polypeptide chains of the hemoglobin molecule. These changes are due to mutations in the coding and non-coding sequences of the (human) alpha- or beta-globin genes. When the synthesis of the alpha- or beta-globin chains is reduced due to mutations, an unbalance between both chains forms the basis of the hereditary microcytic hypochromic anemia. Dependent on the chain with reduced synthesis, these forms of hemoglobinopathies are called alpha- or beta-thalassemia. Hemoglobinopathies may be highly prevalent in geographic areas where malaria tropica is, or has been, endemic, and form an important health issue among the populations living there. Even though previously hemoglobinopathies were rare in the North-European population (with an incidence lower than 0.1%), the frequency of thalassemia alleles has increased dramatically within the last few decades as a consequence of immigration from a large number of countries with a high incidence of severe forms of hemoglobinopathy.

The alpha-globin gene cluster is located on the short arm of chromosome 16, while the beta-globin gene cluster is located on the short arm of chromosome 11. The duplicated alpha1- and alpha2-genes code for the alpha-globin chains during foetal and adult life. In total 4 alpha-globin genes and 2 beta-globin genes are responsible for the production of adult HbA (cr2P2). The loss of one or two alpha globin genes by deletions (with 85% the most common molecular cause of alpha-thalassemia) or point mutations generally results in mild or no clinical complaints. The loss of three functional alpha-genes results in a moderate to severe microcytic hypochromic anemia known as HbH disease. The loss of four alpha-globin genes, a condition known as Hb Bart's Hydrops Fetalis, is not compatible with life and generally leads to severe complication during pregnancy due to a hydropic placenta or fetus and intrauterine death. Even though the majority of alpha-thalassemia cases are due to deletions in the alpha-cluster, an increasing number of point mutations is known to date, interfering with alpha-globin gene expression.

In contrast to alpha-thalassemia, the molecular cause of beta-thalassemia is mainly point mutations interfering with beta-globin gene expression. Several mutants are known to alter the beta-globin chain structure, the most frequent in the world population are HbE, HbC, HbD and HbS. The latter involves an amino acid substitution at position 6 of the beta-globin chain (Glu->Val) and in homozygous form (HbS/HbS) or in compound heterozygous form (HbS/beta-thalassemia, HbS/HbD or HbS/HbC) causes Sickle Cell Disease (SCD). More than 180 mutations in the beta-globin gene and 5' and 3' UTR's are known to cause beta-thalassemia by partial (β⁺-thalassemia) or complete (β⁰-thalassemia) reduction of beta-globin gene expression. Carriers are generally asymptomatic, showing a mild microcytic hypochromic anemia. Homozygotes or compound heterozygotes on the other hand show variable clinical severity from moderate microcytic hypochromic anemia, free from blood transfusion (beta-thalassemia intermedia) to severely anemic, blood transfusion dependent beta-thalassemia Major.

The only cure presently available is Bone Marrow Transplantation, which by the absence of a suitable donor or lack of medical means (the highest prevalence of these diseases are often found in countries with a low standard of medical care) is not always available. Alternatively prenatal diagnosis and selective abortion is offered to couples at risk for these heritable diseases. This can be offered retrospectively to couples already having a severely affected child, or to parents of neonates which have been positively screened for hemoglobinopathy carriership. Another possibility is preconceptional identification of carriers and promoting partner analysis. Whatever choice will be made, a high throughput DNA analysis technique to confirm the hematological diagnosis in carriers and patients and suitable for prenatal diagnosis, is required in the near future. Such a high throughput DNA based approach should be cost effective, reliable.

Allele Specific Oligonucleotide (ASO) hybridization has been widely applied to identify (point) mutations in a variety of disease genes. The short DNA duplexes formed between sample DNA (often PCR amplified DNA) and matching oligonucleotide probes are stable under specific conditions of temperature and salt concentration.

### Detailed description of the invention

The present invention provides a set of probes for detecting a mutant allele of an alpha or beta globin gene, said set comprising at least 10 different oligonucleotides having a continuous stretch of at least 20 and/or at least 21 nucleotides wherein said oligonucleotides comprise between zero and three mismatches compared to the mutant allele which it is intended for. Said oligonucleotides each comprise a sequence specific for (and overlapping) the mutation in the mutant allele. Said sequence specific for the mutation may be flanked on one or both sides by a nucleotide that is not present in either the wild type or the mutant in allele. Each of said oligonucleotides in the set comprises at one end of the continuous stretch at least 5 nucleotides of which at least 4 are specific for a wild type allele. Said at least 5 nucleotides are located at the end but are within the continuous stretch of said 20 or 21- nucleotides. In 21-mer oligonucleotides, these at least 5 nucleotides are located at either end, and within the continuous stretch of the 21 nucleotides. This warrants that the mutation is in the central part of a 20- or 21-mer. In 20-mer oligonucleotides, i.e. having a continuous stretch of 20 nucleotides, a stretch of at least 4 nucleotides, of which at least 3 are specific for the wild type allele is present on one end of (but within) said continuous stretch, while the other end comprises said stretch of 5 nucleotides of which at least 4 are specific for the wild-type allele. In a preferred embodiment, each of said oligonucleotides comprises at either end at least 6 nucleotides of which at least 5 are specific for the wild type allele. The latter embodiment is particularly useful for sets of probes that have a continuous stretch of at least 21 nucleotides. In another preferred embodiment said set of probes comprises at least 11 probes that have a continuous stretch of at least 21 nucleotides. This embodiment is preferred for the embodiment wherein each of said oligonucleotides comprises at either end at least 6 nucleotides of which at least 5 are specific for the wild type allele.

A set of probes of the invention preferably comprises at least 10 oligonucleotides that detect the same strand of the allele. Using oligonucleotides as a probe for the detection of a mutation in a double stranded sample nucleic acid, one has the choice to design an oligonucleotide to be complementary to one strand or the opposing strand. A set of probes preferably comprises at least 10 oligonucleotides that detect the same strand of the allele. In this embodiment said set of probes essentially spans the mutation in the mutant allele. This offers the possibility to select the best probe from the set after a series of tests and include this best probe in a dedicated array that contains probes for other (point)mutations of the alpha and/or beta-globin genes. In a preferred embodiment there are two set of probes specific for the same point-mutation wherein each of the sets recognizes one strand of the double stranded sample nucleic acid.

The sample nucleic acid comprising the alpha- or beta-globin genes to be tested can be single stranded. However, typically the sample nucleic acid is double stranded nucleic acid that is denatured prior to incubation with a set of probes of the invention.

A set of probes of the invention has many advantages. For instance, the multitude of different probes for the same mutation significantly increases the reliability of the assay. A larger number of probes in a set warrants that also under slightly varying conditions a reliable answer can be given. A set of probes of the invention preferably comprises at least one oligonucleotide that can discriminate well between the mutant and the wild type allele, also under difficult circumstances.

It has been observed that differentiation between a wild type allele and a mutant allele using a mutant allele specific oligonucleotide improves significantly by introducing at least one mismatch in said oligonucleotide. Said at least one mismatch is preferably in a part of said oligonucleotide that is not specific for the mutation. In this way said at least one mismatch reduces the hybridisation strength of the probe to the target (mutant) allele and also with a wild type allele. However, the difference in hybridisation of the probe to the mutant allele and the wild type allele can be visualized better, as the wild type allele now has at least two mismatches with the probe. Said at least one mismatch is preferably located in the central part of the oligonucleotide, but it can also be present at either end of the oligonucleotide. A central part of an oligonucleotide is a stretch of nucleotides that encompasses the central nucleotide (21-mer) or nucleotides (20-mer) and at least 4 of nucleotides at either side of the central nucleotide(s). A mutation to be detected is preferably a point mutation, i.e. a mutation wherein the nucleic acid sequence of the mutant and the wild-type allele differs in only one position. In a preferred embodiment, an oligonucleotide of the invention comprises one mismatch when compared with a mutant allele.

The probe can have more nucleotides at either end of the continuous stretch, however, these are typically not derived from either of the globin genes. The additional nucleotides can for instance be added to allow easy coupling to a matrix. Another reason for including such additional nucleotides is to allow the probe to extend further out of the surface to which it is attached thereby allowing easier access of the sample nucleic acid.

In a preferred embodiment an oligonucleotide is specific for a mutant allele as specified in table 1. In table 1, a list of mutations in alpha and beta globin genes is given. In a preferred embodiment of the present invention, the mutant allele, for which the set of probe is made, is an allele of table 1. Therefore, the present invention provides a set of probes as described above, wherein said mutant allele is an allele specified in table 1.

An important application of a set of probes of the invention is the use thereof in a method that can discriminate many different (point)mutations in the alpha- and/or beta-globin gene. Thus the invention further provides a collection of probes comprising at least 20 different sets of probes of the invention. This collection is capable of detecting a variety of mutations and can be used to screen patients from various parts of the world, i.e. where different mutations are prevalent.

An oligonucleotide (oligo-probe) of the invention can be constructed in a sense or in an anti-sense direction. Thus a probe can detect an allele in the sense strand or in the antisense strand configuration. In one embodiment, the present invention provides a set of probes, wherein each of said oligonucleotides detects the same strand of the allele. Said mutations may comprise single nucleotide polymorphisms or SNPs, or they may comprise deletions or insertions of one or more nucleotides. Therefore, the present application provides a collection of probes comprising at least 20 different sets of probes as described above.

In a preferred embodiment, a collection of probes comprises at least two set of probes of the invention. More, preferably, said collection comprises at least five sets of probes of the invention. More preferably, at least 20 sets of probes. In a particularly preferred embodiment a collection of probes comprises set of probes for at least the different alpha-globin mutant alleles specified in table 1. Preferably, said collection comprises sets of probes of the invention for 42 different alpha-globin mutant alleles as specified in table 1.

The present application further provides a collection of probes comprising sets of probes for at least 40 different beta-globin mutant alleles as specified in table 1. More preferably, said collection comprises set of probes of the invention for all of the beta-globin mutant alleles specified in table 1. Preferably, said collection of probes comprises set of probes of the invention for 66 different beta-globin mutant alleles specified in table 1.

An collection of probes covering most mutations causing alpha- and beta-thalassemia world-wide is provided. Such a preferred collection of probes is applicable in the diagnosis of thalassemia in patients from most countries in which thalassemia is endemic and causes a serious health problem.

The invention further provides a collection of probes, comprising two different sets of probes for detecting the same mutant allele and wherein one set of probes is specific for one strand and wherein the other set of probes is specific for the opposite strand. In one embodiment a collection of probes according to the invention thus comprises two set of probes of the invention wherein one set of probes is specific for the sense strand of a mutant allele and the other set of probes is specific for the anti-sense strand of the same mutant allele. In a further preferred embodiment the invention provides a collection of probes according to the invention, wherein said collection further comprises a probe for detecting the corresponding wild type allele of at least one of the mutant alleles for which said collection comprises a specific probe. A probe specific for the corresponding wild type allele is a probe for about the same part of the sequence as the corresponding mutant allele specific probe but that at least differs from said mutant allele specific probe in that said wild type allele specific probe is specific for the wild type sequence at the position of said mutation.
A collection of probes according to this embodiment of the invention, if used in a diagnostic test, is capable of giving a detection signal for a mutant allele and a detection signal for the corresponding wild type allele. Comparing the detection signal for a mutant allele to a detection signal of a wild type allele enables one to more clearly distinguish between a mutant, abnormal, status and a wild type, normal, status. Furthermore one is able to more reliably predict whether a mutant is homozygous or heterozygous. Therefore, a diagnostic test can be performed under variable conditions while having a greater certainty of stable results of different experiments as compared to the embodiment wherein a wild type allele is not simultaneously detected. The greater certainty of stable results is, among others, caused by the fact that the value for a detection signal of a wild type allele is determined under the same conditions as the value for a detection signal of a mutant allele in this embodiment. Alternatively, a detection signal obtained with an oligonucleotide specific for a mutant allele is related to a reference value. Reference values are preferably determined for a wild type detection signal, a heterozygous mutant detection signal and/or a homozygous mutant detection signal. When relying on reference values, a test is preferably performed under conditions that are commensurate with the circumstances for which the reference values are applicable. An example of conditions for determining reference values is given in the examples.

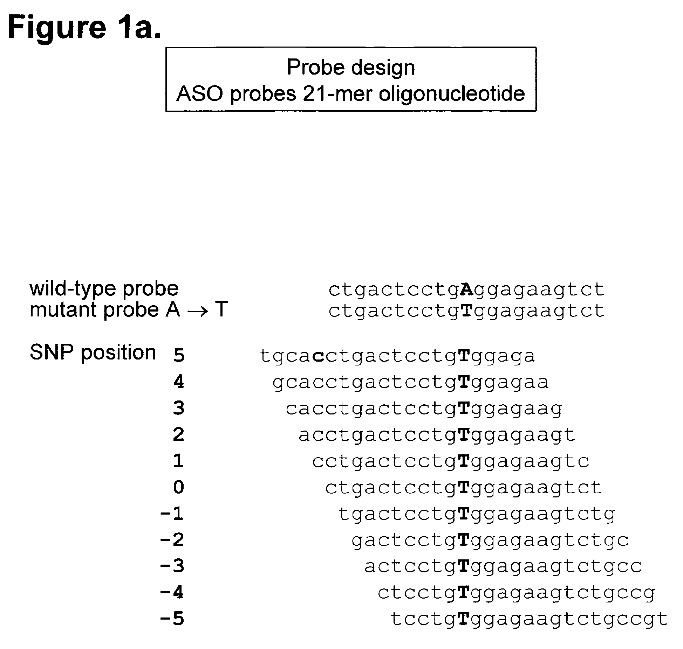

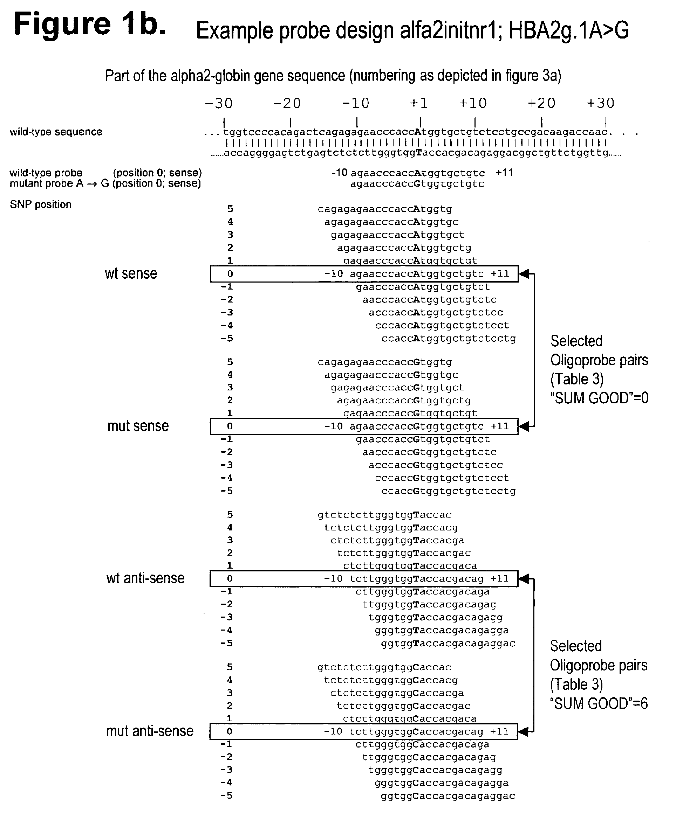

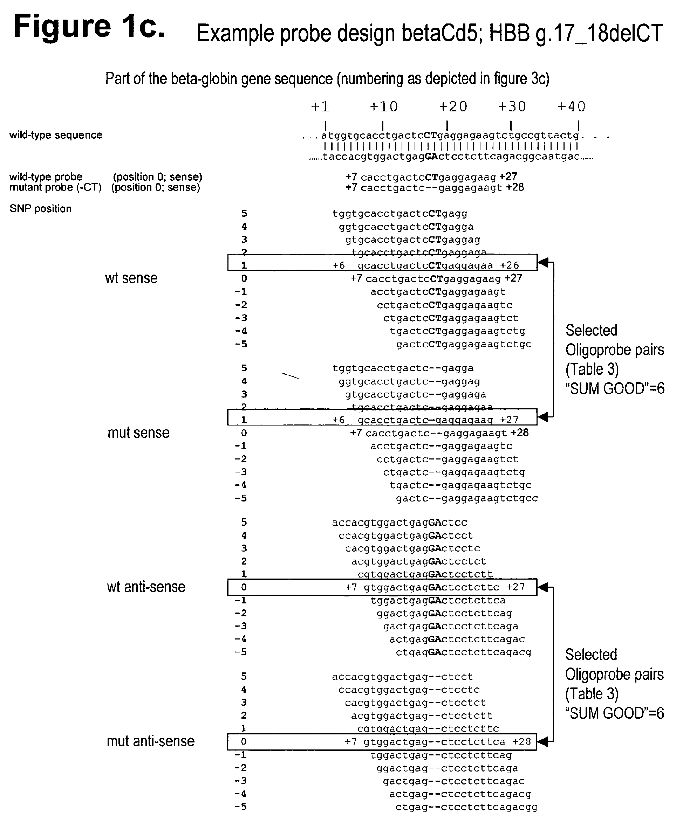

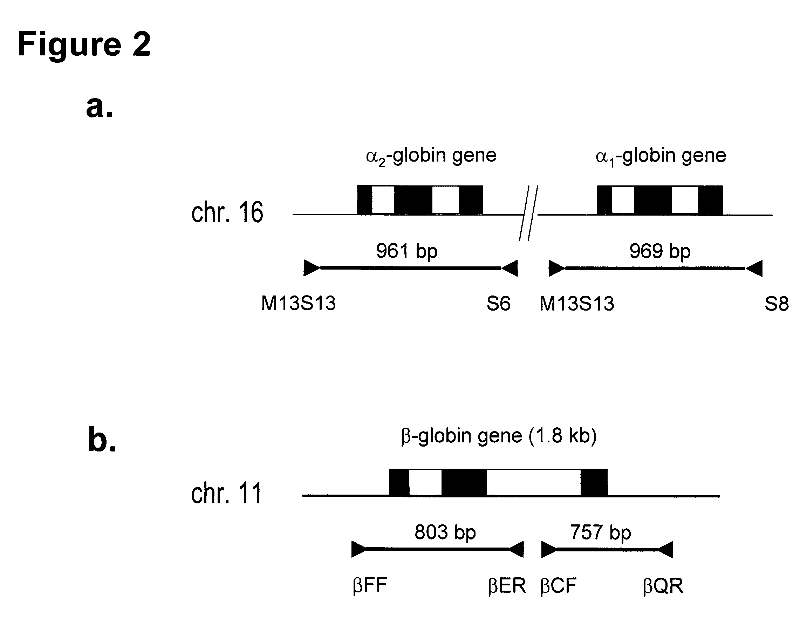

Table 2 shows an experiment wherein six identical arrays were hybridized with a mix of 4 PCR fragments covering the complete beta- and alpha-globin gene sequences. Schematic presentations of the primer positions for PCR for the alpha 1 globin gene, the alpha 2 globin gene and the beta-globin gene are shown in figure 2. Each mix contained besides the wild type sequences also a different combination of mutations as indicated in Table 2. Oligonucleotide probes for the detection of mutations were made according to the example of figure 1. From each of these six experiments a data set was obtained consisting of signal intensities for each of the 11 wild type sense and anti-sense and mutant sense and anti-sense oligo-probes tiling up from +5 to - 5 with respect to the SNP position 0. The sensitivity for each probe pair was determined by the ratio of wild type signal divided by the sum of the wild type and mutant signal (wt/(wt+mut)). If the ratio was higher than 0.75, the SNP was typed as "normal", if the ratio was between 0.25 and 0.75 the SNP was typed as heterozygous, if the ratio was below 0.25 it was considered homozygous for the presence of the mutation. For each of the 11 oligoprobe pairs for which the SNP was typed correctly a value of 1 was added up for each experiment. Thus the highest score is 6, being the sum of all experiments in which the oligoprobes could distinguish between wild type and mutant (or non-wild type). This is used as a measure for the sensitivity of each probe pair.

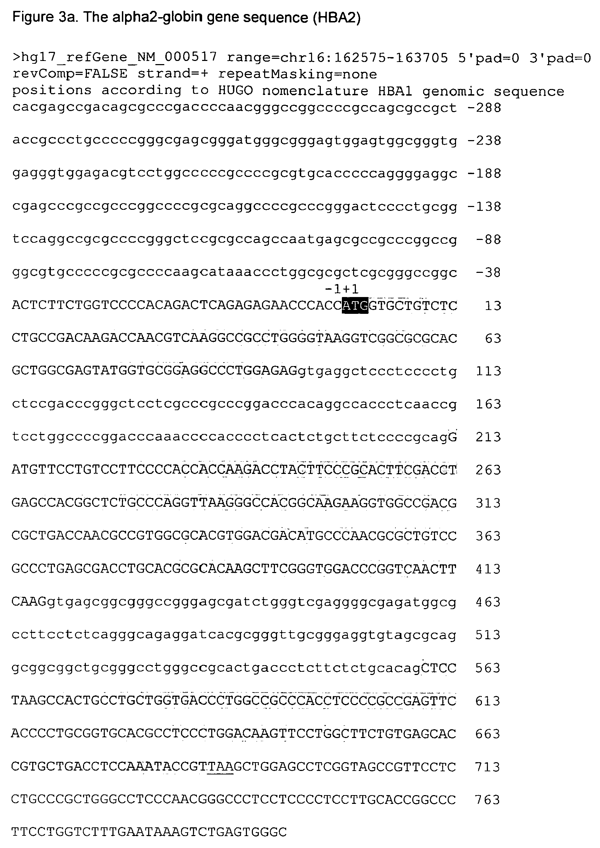

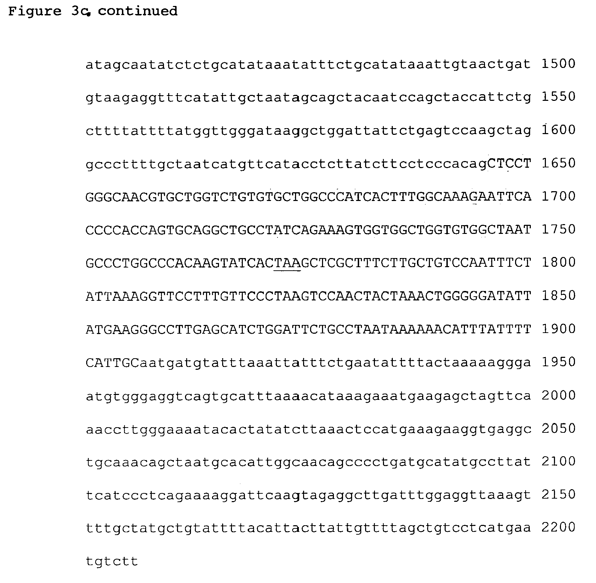

From the data obtained from the 6 experiments as depicted in table 2, a selection was made of 2 oligo-probe pairs (wild type and mutant, sense and anti-sense) which had the highest score per oligonucleotide (SNP). These data are shown in Table 3, which comprises a list of oligo-probes that are selected for each mutation or SNP. The SNP position is given from the centre (0) and the primer sequence can be determined from the first and last nt position in the corresponding gene sequence, which are shown in Figure 3a to c. The selection was based on the sum of the highest scores in the 6 experiments performed (SUMGOOD=sum of experiments in which the SNP is scored correctly). If probes showed equally high sensitivity, the probe with it's SNP position nearest to the center (oligo-probe position 0) was selected. If, however, the result appeared to be an incorrect score for the presence (or absence) of the mutated SNP a value of 1 was added up in the column "SUMWRONG" indicating in how many cases the oligoprobe pair failed to resolve mutant from wild type. If the signal was outside the reliable range, no ratio could be determined and a value of 1 per experiment was added up in the column "SUMND" (sum not determined).

Preferred oligonucleotides of the invention are listed in table 3. Thus, in a preferred embodiment the invention provides a collection of probes, comprising at least one oligonucleotide specific for an alpha-globin mutant allele or a beta-globin mutant allele of table 3. In a preferred embodiment a collection of probes according to the invention comprises at least two oligonucleotides specific for an alpha-globin mutant allele or a beta-globin mutant allele of table 3, preferably at least 5, more preferably at least 20, more preferably at least 216 different mutant allele specific probes of table 3. A larger number of probes increases the reliability of the diagnostic test. A collection that is specific for a larger number of different mutations is capable of detecting more types of mutations. Comprising more different oligonucleotides in a collection of probes that are specific for the same mutation increases the probability and reliability of detection of that specific mutation. In a preferred embodiment the invention provides a collection of probes according to the invention, wherein said collection further comprises a probe for detecting the corresponding wild type allele of at least one mutant allele. In a more preferred embodiment the collection of probes according to the invention comprises probes for detecting the corresponding wild type alleles of at least two mutant alleles, preferably at least 5, more preferably at least 20, more preferably said collection of probes comprises probes for at least 216 of the corresponding wild type alleles. Preferably, said collection of probes comprises a corresponding wild type specific probe for each mutant allele specific probe of table 3 in said collection. Preferably, said corresponding wild type specific probe is a wild type specific probe of table 3.

Preferred oligonucleotides of the invention are listed in table 3. A collection of probes of the invention therefore preferably comprises at least one oligonucleotide specific for a mutant allele as specified in table 3. in one aspect the invention provides a collection of probes for the detection of mutations in a alpha- or beta globin gene said collection of probes comprising at least one, preferably at least two, preferably at least 5, and more preferably at least 20 different oligonucleotides specific for a mutant allele depicted in table 3. Preferably said collection of probes comprises mutant allele specific oligonucleotides of table 3 having a score of at least 1 for Sum Good. A score of 1 for Sum Good in table 3 means that the oligonucleotide correctly distinguished the wild type signal from the mutant signal in 1 experiment as depicted in table 2. Preferably said collection of probes comprises oligonucleotides having a Sum Good score of at least 2, more preferably, a score of 6. Preferably, a collection of probes comprises at least two oligonucleotides having said Sum Good score. Preferably said collection of probes comprises at least 5, more preferably at least 20 and more preferably at least 200 mutant allele specific oligonucleotides of table 3. In a particularly preferred embodiment the invention provides a collection of probes comprising essentially all mutant allele specific oligonucleotides of table 3. In a preferred embodiment the invention a collection of probes comprises a mutant allele specific oligonucleotide of table 3 having a score of 0 for Sum Wrong. A score of 0 for Sum Wrong in table 3 means that there was no experiment (as depicted in table 2) wherein the oligonucleotide failed to resolve mutant from wild type. In a preferred embodiment the invention provides a collection of probes according to the invention, comprising at least 5, preferably at least 20 mutant allele specific oligonucleotides of table 3 having a Sum Wrong score of 0.

As already mentioned, it is better to be able to make a diagnosis based on the screening of at least 42 different alpha-globin mutant alleles. Therefore, the present invention discloses in a preferred embodiment, a collection of probes according to the invention, comprising probes for at least 42 different alpha-globin mutant alleles specified in table 3. Testing for at least 40, more preferable at least 66 mutations in the beta-globin gene in addition to the alpha-globin gene would increase the value of the diagnostic screening test. Therefore, the present application also provides a collection of probes as described above, comprising probes for at least 40, most preferably 66 different beta-globin mutant alleles specified in table 3.

The invention further provides a collection of probes according to the invention, comprising at least two different probes of table 3 for detecting the same mutant allele and wherein the oligonucleotide in one probe detects one strand of the allele and wherein the oligonucleotide in the other probe detects the other strand of the allele. Such a collection of probes is capable of detecting a sense and an anti-sense strand of a mutant or a wild type allele. Thereby increasing the chance of detecting a mutant allele, or correctly assessing the presence of a wild type allele.

A collection of probes according to the invention is preferably used to screen a sample for the absence or presence of a specific mutation as listed in table 1. However, considering that the probes span a considerable region of the genes tested, it is also possible to detect deletions other than those listed in table 1, as such deletions will likely overlap with one or more of the mutant allele specific probes in the collection. Such mutations can thus also be detected.

The above described collections of probes are useful for the production of array in all kinds of formats, for example homogeneous test formats or heterogeneous test formats, i.e. tests based on a solid phase. Homogeneous formats lack separation steps and therefore allow the genotyping reactions to be performed and analysed in the same test tube. There is a wide range of different heterogeneous assays, most of which apply solid phase immobilization of target, template or a generic identification sequence. Examples of solid phase tests are microtiter plates and microarrays.

Microarray comprises a miniaturized solid-support format carrying sets of ordered, immobilized molecules, which facilitate parallel analysis of reactions. An example of a solid support can be a glass surface, nylon membranes, magnetic beads, polyacrylamide gel, gel pads or silicon chips. In general, the probes arranged in the microarray on a chip combine with complementary sequences present in the sample.

Therefore in another embodiment, the invention provides an array comprising at least one collection of probes as described above. Bound sequences can be detected because the sample was "tagged", meaning that some sort of detection moiety was bound to the sequences in the sample. Said detection moiety may comprise for example a fluorescent molecule, or a chemical group which is visualized in some chemical reaction, or a molecule that gives a signal once it is combined with other compounds, or a radioactive signal or any other moiety that is detectable in any way. Therefore, in yet another embodiment, the invention provides a kit of parts, comprising at least one collection of probes as described above and a means of detection.

Because the genes encoding alpha and/ or beta globin are comprised in the genomic DNA of an individual, said collection of probes is useful for determining mutations in the alpha and/ or beta globin genes in a sample of DNA. Such a sample can be obtained from a human individual by a sample of blood or tissue, or any other bodily sample that contains DNA originating from the body of said human individual. Therefore, the present invention in another embodiment teaches the use of a collection of probes as described above and/or an array as described above, and/or a kit of parts as described above for determining mutations in alpha and/ or beta globin genes in a sample of DNA. Because mutations in alpha and/ or beta globin genes in a sample of DNA may give rise to a hemoglobin-related disorder, or hemoglobinopathy, the present invention teaches the use as described above for determining the presence of a hemoglobinopathy gene pattern in a sample of DNA.

Thalassemia related mutations in DNA are hereditary, which means that the combination of genes from the mother and father may cause homozygote presence of mutated alleles, in which case the child may suffer from thalassemia in a more severe form. Screening both parents DNA for the occurrence of mutations in alpha and/ or beta globin genes is useful in determining the risk or the increased risk of the child to develop thalassemia. Therefore, the present application teaches the use as described above, for determining an increased risk of developing a blood related disorder. Furthermore, the present application teaches a method for predicting an increased risk of developing an hemoglobinopathy in the progeny of a human individual comprising the steps of:
- contacting a sample of DNA of said individual with a collection of probes as described above and/or an array as described above, and/or a kit of parts as described above;
- measuring binding of said sample of DNA to said collection of probes, and/or said array, and/or said kit of parts;
- determining the presence of one or more mutations of an alpha or beta globin gene in said sample of DNA.

For the above-described method, it is sometimes useful to amplify the genes of interest in order to obtain a better result in the methods as described above. Said amplification is usually done by PCR or a related technique. Therefore, the present invention also teaches a method as described above, which includes an amplification step.

An oligonucleotide of the invention can be unmodified or comprise a modification as long as the hybridisation characteristics are not modified in kind. The hybridisation characteristics may be modified in amount, although they are preferably not modified. Derivatives and/or analogues of oligonucleotides of the invention can therefore also be used to replace or in addition to oligonucleotides of the invention. Non-limiting examples of such derivatives and/or analogues are Locked nucleic acid (LNA), peptide nucleic acid (PNA), morpholino's and other derivatives and analogues. An oligonucleotide of the invention can also comprise nucleotide analogues having the same base-pairing characteristics in kind, not necessarily in amount, as the nucleotide(s) they replace. A mismatch preferably comprises a nucleotides having a different base-pairing characteristic in kind and amount than the nucleotide it replaces. However, alternatively, the mismatch is a nucleotide analogue having a different base-pairing characteristic in kind and amount than the nucleotide it replaces.

The invention is further explained in examples in the experimental part, without being limited to it.

### Examples

Micro-arrays are generated by in situ oligonucleotide synthesis using a light activated process employing a digital micro mirror device and highly efficient photochemistry as described by Baum et al. (2003).

Both frequently occurring and rare mutations of the alpha- and beta-globin gene causing abnormal hemoglobins, alpha- or beta-thalassemia were selected from literature (Huisman et al 1998, Bernini & Harteveld 1998)(Table 1). Oligo-probes were designed for the detection of in total 66 beta- and 42 alpha-thalassemia mutations. Oligo-probes were designed as 20- or 21-mers for which the SNP-position was located in the middle of the probe (position 0). Subsequent probes were defined shifted respectively 1 to 5 nucleotides to the left (position +1 to +5) or to the right (positions -1 to -5) for both sense and anti-sense containing the wild type and the mutant sequence (an example is shown in figure 1). Because mutations are clustered both in the alpha and beta-globin genes, a number of the wild type probes are shared by different mutation specific probes. The total number of sense and anti-sense probes for the 66 beta-globin gene specific SNP's are 2184 and for the 42 alpha-globin gene specific SNP's 1484 probes.

To characterize mutations of the alpha- and beta-globin genes, primers were selected for amplification. The duplicated alpha-globin genes (GenBank HUMHBA4; alpha1-globin gene 10400-11357, alpha2-globin gene 6589-7550) were selectively amplified using the alpha2-globin gene specific reverse primer S6 (5'-TCCATTGTTGGCACATTCC-3') or the alphal-globin gene specific reverse primer S8 (5'-CTGGCACGTTTCTGAGGGAA-3'), located in the 3' UTR of these genes. The forward primer M13S13 (5'-CGACGTTGTAAAACGACGGCCAGTCGCCAGCCAATGAGCGCC-3'), located in the 5'UTR, is identical for both duplicated genes. The amplification products are 962 bp for the alpha2-globin gene (M13S13 and S6) and 958 bp for the alphal-globin gene (Figure 2a). The beta-globin gene was amplified in two fragments using primer combinations beta FF (5'-TGAAGTCCAACTCCTAAGCCA-3') and beta-ER (5'-AAACGATCCTGAGACTTCCA-3') (5' fragment; GenBank U01317 61965-62767) and beta-CF (5'-GTGTACACATATTGACCAAA-3') and beta-Q (5'-AAATGCACTGACCTCCCACA-3')(3' fragment; GenBank U01317 63088-63844). The 5' fragment is 803 bp long and contains the 5'UTR, exon 1, intron 1, exon 2 and part of intron 2, while the 3' fragment is 757 bp in length, containing a part of intron 2, the complete exon 3 and part of the 3'UTR (Figure 2b.).

The control templates used for hybridization were genomic DNA samples of carriers and patients, homo- or heterozygous for 8 different mutations in the beta-globin gene and 7 mutations in the alpha-globin genes. Six identical micro-arrays were hybridized with different PCR products as indicated in Table 2. Columns represent the 6 different experiments (numbered 1 to 6), the rows indicate the PCR fragments used as templates for hybridization. The cells show the mutated alleles, either heterozygous or homozygous, present in the equivalent PCR fragments for each experiment.

Genomic DNA (100 ng) was amplified in 50 µl using 10 pmol primers (beta-FF + betaER or Beta-CF + betaQ), 1 Unit AmpliTaq polymerase (Perkin-Elmer-Cetus) and 5 µl of 10xbetaPCR buffer (500 mM KCL, 100 mM Tris HCl pH 8.4, 25 mM MgCl2, 2 mg/ml BSA, 2 mM dNTP's). This mixture was heated at 94°C for 30 sec., 65°C for 60 sec. and 72°C for 120 sec. on a Stratagene Robocycler 40. The PCR was concluded by an incubation at 72°C for 6 min. Five µl of PCR product was analyzed on a 1% agarose gel. The amplification for the alpha-globin genes contains 200 ng of genomic DNA in 50 µl using 10 pmol primers (M13S13 + S6 for alpha2 and M13S13+S8 for alpha1), 5 µl of 10x alphaPCR buffer (500 mM KCL, 200 mM Tris HCl pH 8.4, 15 mM MgCl2, 2 mM dNTP's) and 1 mol/l betaine. This mixture was heated 8 minutes at 95°C, 2.5 Units of Platinum Taq polymerase (Life Technologies, Gaithersburg, MD) was added, followed by 30 cycles of denaturation at 94°C for 45 sec., annealing at 60°C for 75 sec. and extension at 72°C for 150 sec. and 5 cycles of 94°C for 45 sec., annealing at 60°C for 75 sec and extension at 72°C for 300 sec. (Stratagene Robocycler 40).

The amplification products were purified using the QIAquick®PCR Purification Kit (Qiagen cat. No. 28106, QIAGEN GmbH, D-40724, Hilden, Germany) and dissolved in 60µl of H20 to a final concentration of approximately 50 ng/µl.

The PCR products were digoxygenated by random priming according to a modified protocol by Baum et al. (2003) using 5.4 µg DNA in 52.8 µl DEPC-H₂O. After denaturation for 5 minutes, hexamers were added to a final concentration of 22.5 ng/µl. Subsequently 24.2 µl of a reaction mix was added, containing 200 mM Hepes pH6.6, 50 mM Tris-HCl pH 8.0, 5 mM MgCl₂, 10 mM 2-Mercaptoethanol, 50 µM of each dATP, dGTP and dCTP and 32.5 µM dTTP, 17.5 µM DIG-dUTP, 0.4 mg BSA/ml and 7.5 U of Klenow fragments to a final volume of 80µl and incubated at 37°C for 3 hours on a thermomixer. The labeled DNA fragments were fragmented by adding 3.0 µl of a DNaseI solution of 0.5 U/µl, followed by incubation at 37°C for 30 seconds. The DNase was inactivated by heating at 99°C for 15 minutes. The labeled DNA fragments were precipitated by adding 0.1 volume 3 M Sodium Acetate pH5.6 and 2.5 volumes of absolute ethanol. The pellet was dried by speedvac centrifugation and dissolved in 5 µl DEPC-H₂O. Subsequently 15 µl of the hybridization solution, containing 101.25 mM Morpholinoethansulfonic acid pH6.5-6.7, 0.9 M NaCl, 20 mM Na2EDTA and 0.01% (v/v) Tween 20, was added. After 3 minutes of denaturation at 95°C, the sample was put on ice to be used immediately for array hybridization.

Detection and feature readout were performed using the CCD-based detection system of the geniom device (Cy3 filter set) as described by Baum et al. (2003). Signal intensities are expressed in counts with the linear range of the CCD being 0 to 65000 counts. Intensities higher than 60000 counts are considered saturated and are discarded from further calculations. Six identical arrays were hybridized with a mix of 4 PCR fragments covering the complete beta- and alpha-globin gene sequences. Each mix contained besides the wild type sequences also a different combination of mutations as indicated in Table 2. From each of these six experiments a data set was obtained consisting of signal intensities for each of the 11 wild type sense and anti-sense and mutant sense and anti-sense oligo-probes tiling up from +5 to - 5 with respect to the SNP position 0. The sensitivity for each probe pair was determined by the ratio of wild type signal divided by the sum of the wild type and mutant signal (wt/(wt+mut)). If the ratio was higher than 0.75, the SNP was typed as "normal", if the ratio was between 0.25 and 0.75 the SNP was typed as heterozygous, if the ratio was below 0.25 it was considered homozygous for the presence of the mutation. For each of the 11 oligoprobe pairs for which the SNP was typed correctly a value of 1 was added up for each experiment. Thus the highest score is 6, being the sum of all experiments in which the oligoprobes could distinguish between wild type and mutant (or non-wild type). This is used as a measure for the sensitivity of each probe pair.

### Results

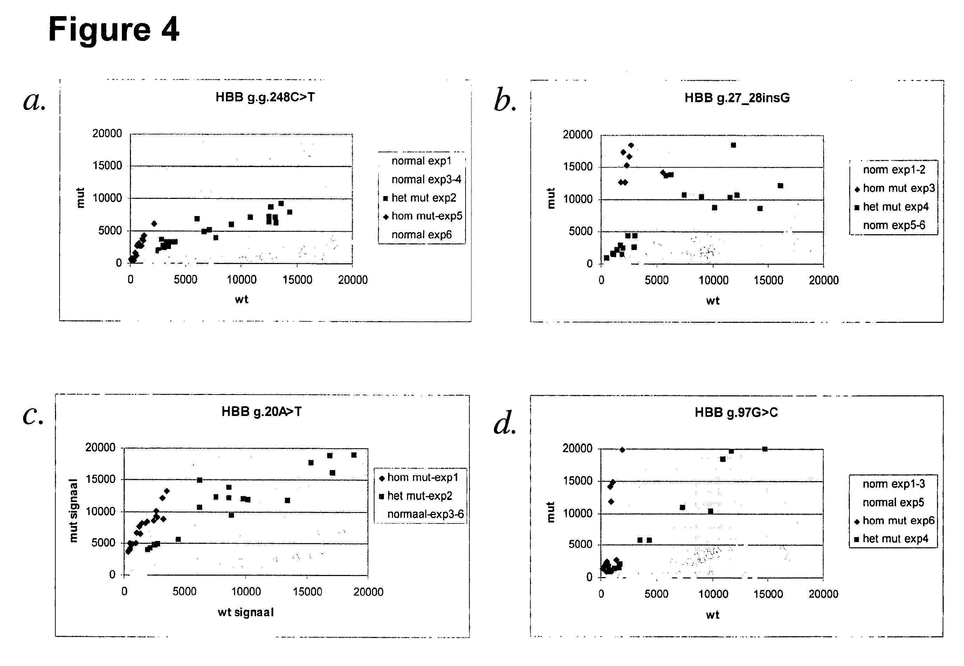

Eight different beta-globin gene mutations were used as positive controls in 6 experiments to test the ability of the system to detect the mutated allele (Table 2). Six out of eight (HBB g.20A>T, g.79C>T, g.27_28insG, g.97G>C, g.1424+22A>G and g.1190C>G) were identified correctly, including the 5 heterozygotes and 4 homozygotes. However 1 was scored as homozygous while heterozygous (experiment 3; HBB g.1344G>T) and for 1 case the signal appeared too low (experiment nr 4; het. HBB g.1424+20A>G). Figure 4 shows examples of wild type, hetero- and homozygotes for some of the beta-gene mutations used as positive controls. The signal intensities of wild type and mutant both sense and anti-sense are put on the X and Y axis respectively, the clustering of data points shows a clear distinction between wild type (clustering around the X-axis), homozygosity (clustering around the Y-axis) and heterozygosity (clustering diagonal) for the HBB g.248C>T, g.20A>T, g.97G>C and g.27_28insG mutations.

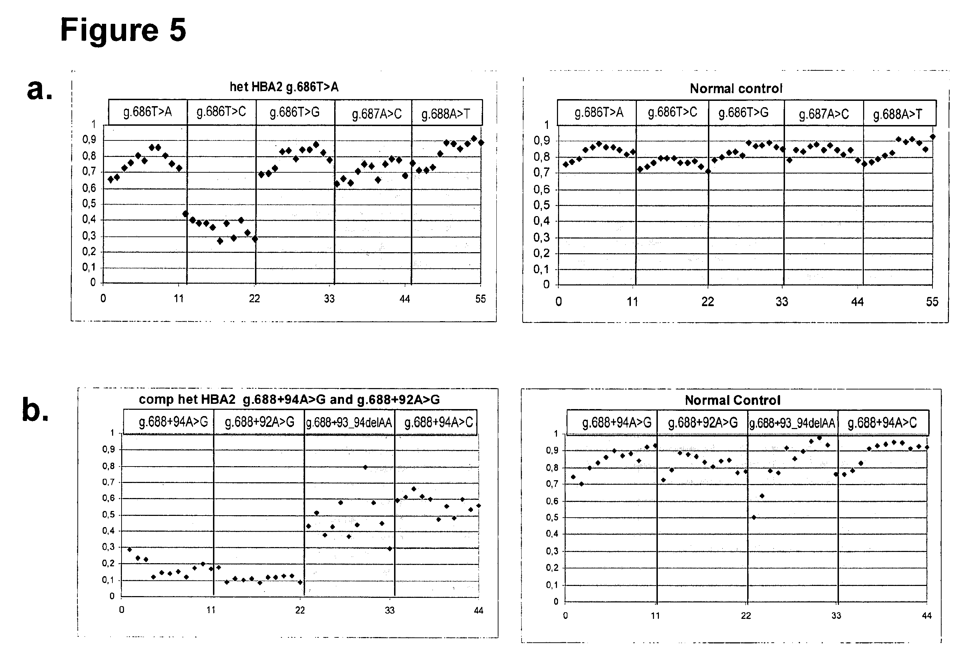

Similarly 7 different alpha-thalassemia mutations were tested in experiment 1 to 6 (as outlined in Table 2), all of which were assigned correctly by sense and anti-sense oligoprobe pairs. Even though the HBA1 g.624C>T anti-sense oligoprobe set failed because of low signals in experiment nr 3, the sense oligo-probe set identified this mutation correctly as heterozygous in the same experiment. Two examples of the outcome of experiments 1 and 5 are shown in figure 5. The pattern of ratio's for oligoprimer pairs +5 to -5 as seen in figure 5 shows a curve with an optimum towards the middle of the probe, even though in the majority of cases, the optimum is not exactly in the center of the probe, but shifted up- or downstream from the middle, which indicates that a centered position for the SNP is not necessarily the best performing Allele Specific Oligoprobe.

From the data obtained from 6 experiments a selection was made of 2 oligo-probe pairs (wild type and mutant, sense and anti-sense), which had the highest score per SNP (Table 3). The selection was based on the sum of the highest scores in the 6 experiments performed (SUMGOOD=sum of experiments in which the SNP is scored correctly). If probes showed equally high sensitivity, the probe with its SNP position nearest to the center (oligoprobe position 0) was selected. If, however, the result appeared to be an incorrect score for the presence (or absence) of the mutated SNP a value of 1 was added up in the column "SUMWRONG" (Table3) indicating in how many cases the oligoprobe pair failed to resolve mutant from wild type. If the signal was outside the reliable range, no ratio could be determined and a value of 1 per experiment was added up in the column "SUMND" (sum not determined).

Both in the alpha- and the beta-globin genes mutations are clustered and different SNP's can be present at the same position in the gene. It is of particular interest to study the effect of cross-hybridization and possible false positive (or negative) signal in oligoprobes designed to identify different SNP's in overlapping sequences. The oligoprobe sets designed to identify 5 different SNP's around the stopcodon of the alpha2-globin gene are shown in figure 5a. Each set of 11 anti-sense oligo-probes was designed to identify another SNP, differing by as little as a single nucleotide at the same position or by two positions in between. In spite of the sequence overlap in mutation specific oligoprobes, no cross hybridization between the template and almost identical mutant probes was observed suggesting that the specificity is extremely high. The HBA2 g.686T>C specific oligoprobe pair showed a decrease in ratio to 0.5, as expected from the heterozygosity of this allele in the sample tested.

Two mutations located only 2 nt apart on different alleles will lead to the absence of a wild type signal and two independent mutant signals in a compound heterozygote individual as tested in experiment 5 (Figure 5b). The ratio's of the oligoprobe pairs for HBA2 g.688+94A>G and HBA2 g.688+92A>G were close to "0" suggesting homozygosity for both mutations. This was caused by the absence of the wild type allele in this sample. Clustering of SNP's in the same genomic region therefore does not seem to interfere with the capacity to distinguish between the two mutated alleles, which makes the system extremely suitable for the detection of thalassemia mutations, which are characterized by multiple mutations at the same loci in the gene (Thein et al. 1998, Bernini and Harteveld 1998).

From the data shown in Table 3 it appears that for 52 out of 66 SNP's of the beta-globin gene the wild type signal is higher than the mutant signal for both sense and anti-sense oligo-probes. In 11 out of 66 SNP's at least one oligopair (sense or anti-sense) was able to distinguish wild-type from non-wild-type. For 3 SNP's no discrimination between wild-type and non-wild type could be made. The ratio wt/(wt+mut) being higher than 0.75 suggests that the selected oligo-probe pairs will be suitable for mutation detection in 96% of the beta-globin gene SNP's. Similarly 27 out of 42 SNP's in the alpha-globin genes a distinction could be made between wild type and mutant signal in both sense and anti-sense probe pairs, in 13 either the sense or the anti-sense did and for 2 SNP's no suitable primer pair was found. Thus 95% of selected SNP's in the alpha-globin genes could be detected using the selected primer pairs shown in Table 3. This selection of oligoprobe pairs can be used in a more concise microarray format in the future for example for large scale high throughput post- and neonatal screening programs aiming at prevention of hemoglobinopathies.

### Description of the tables

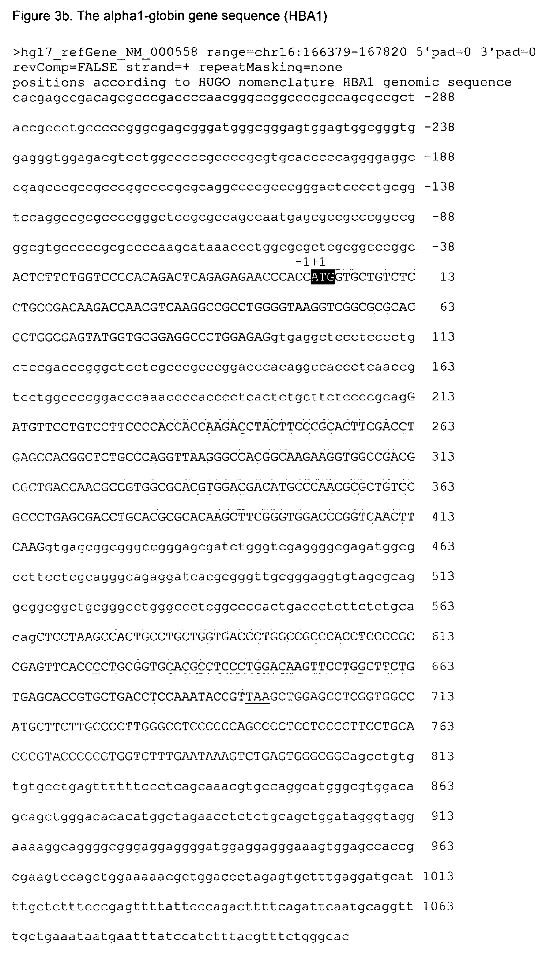

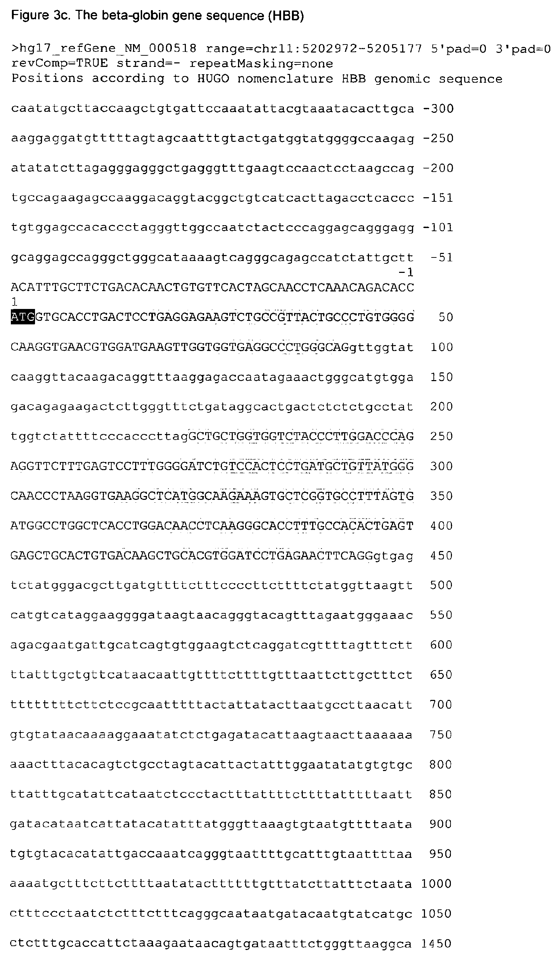

**Table 1** List of mutations in the α- and β-globin gene causing thalassemia or abnormal hemoglobins. The first column represents the oligo names. In the second column the mutation names are given according to the international HUGO-nomenclature consisting of the gene code (HBB for the beta-globin gene, HbA1 and HbA2 for the alpha1- and alpha2-globin genes respectively), the "g." to indicate the genomic sequence, the position of the SNP followed by the mutation (http://globin.cse.psu.edu/cgd-bin/hbvar). The positions for the mutations and the oligoprimers are as indicated in figure 3 a to c for the corresponding genes.
For each mutation a series of 44 oligoprobes are designed, 11 wild type, 11 mutant, both sense and anti-sense. The oligoprobe in which the SNP position is centred in the middle is labelled "0", from this position the primer is designed shifted 1 to 5 nt to the left and to the right labelled -5, -4, -3, -2, -1, 0, 1, 2, 3, 4, 5, where the number indicates the position of the SNP from the middle of the oligoprimer. The sequence of the primer with SNP position "0" is indicated as position numbers corresponding to the sequence in figure 3.

**Table 2** Experimental design. Six identical micro-arrays were hybridised with different PCR products as indicated in the table. Columns represent the 6 different experiments (numbered 1 to 6), the rows indicate the PCR fragments used as template for hybridisation. The cells show which mutated alleles, either heterozygous or homozygous, are present in the equivalent PCR fragments for each experiment.

**Table 3** List of oligo-probes selected for each mutation or SNP. The list shows the best performing wild type and mutant, sense and anti-sense oligoprobe for each mutation. These probes were selected based on the results of 6 experiments. The criteria were determined by the highest sum of the correctly scored wild type signal (SUMGOOD) and the lowest score of false positive signal (SUMWRONG). The SUMND indicates the number of times the results were unclear (Not Determined). The SNP position is given from the centre (0) and the primer sequence can be determined from the first and last nt position in the corresponding gene sequence (Figure 3a to c). If the score is non-decisive (none is preferred) the probe at position 0 was chosen (mutation positioned in the middle of the probe).

### Description of the figures

**Figure 1.** 1A. Example of the oligonucleotide probes made for the detection of the HbS mutation (beta codon 6 GAG>GTG). For every SNP 11 oligonucleotides covering the wild type, the mutant both sense and anti-sense was made according to this scheme. 1B Example probe design alfa2initnrl; HBA2g.1A>G. 1C Example probe design betaCd5; HBB g.17_18delCT.
**Figure 2.** Schematic presentation of primer position for PCR for a. the alpha 1 and alpha 2 globin genes and b. for the beta-globin gene.
**Figure 3.** Human alpha- and beta-globin gene sequences, numbered according to the international HUGO-nomenclature for genomic sequences. Position +1 is the first basepair of the initiation codon. The exons are indicated in capital letters marked by a grey box. Each of the alpha- and beta-globin genes contains 3 exons and 2 introns. The numbers correspond to the mutation names according to the HUGO nomenclature and the primer positions as indicated in Table 1 and 2.
   a. The alpha2-globin gene sequence (accession number NM000517)
   b. The alpha1- globin gene sequence (accession number NM000558)
   c. The beta-globin gene sequence (accession number U01317)
**Figure 4.** Example of graphs showing the distinction between wild type, heterozygote and homozygote for the IVS1-5, cd39, HbS and cd8/9 mutation. The X-axis shows the intensity of the wild type probe signal, both sense and antisense, the Y-axis shows the mutant signal (sense and antisense oligoprobe signal intensities).
**Figure 5.** Example of mutation detection in the alpha-globin gene. The graph shows the signal ratio's wt/(wt+mut) of 11 anti-sense oligoprobe pairs with the SNP position varying from -5 to +5 with respect to the center (SNP oligoprobe pair 0). a. Five SNP sites are shown, clustering around the termination codon 142 of the alpha2-globin gene. The SNP position and mutation are indicated above each set of 11 probes designed to identify the corresponding SNP. The graph on the left represents the results of experiment 5, while on the right the results of a wild type fragment are shown. Heterozygosity for the HBA2 g.686T>C is evident from the ratio decreasing towards 0.5.
   b. Results for SNP analysis between position HBA2 g.688+92 to +94 of a compound heterozygous individual in experiment 5 in comparison to a normal individual. Compound heterozygosity for two mutations in the polyAdenylation site of the alpha2-globin gene (HBA2 g.688+92A>G and +94A>G) leads to the total absence of wild type allele, which results in a homozygous ratio for both SNP's mutated in this individual and unreliable ratio's due to low signal intensities in the two overlapping oligoprobe sequences specific for the neighboring SNP-sites.

### References

- Baum M., Bielau S., Rittner N, et al. Validation of a novel, fully integrated and flexible microarray benchtop facility for gene expression profiling. Nucleic Acids Research, 2003, Vol.31, No23 e151
- Bernini L.F. & Harteveld C.L. Alpha-thalassaemia. In Bailliere's Clinical Haematology, 1998, Vol. 11, No.1, pp.53-90
- Huisman Th. J., Carver M., Baysal E. A Syllabus of Hemoglobin Variants. 1998,
- Thein S.L. Beta-thalassaemia. In Bailliere's Clinical Haematology, 1998, Vol. 11, No.1, pp.91-126

**Table 1 List of mutations for which oligoprobes are designed**

| oligo name | HUGO nomenclature (mutation name(SNP)) | wt/mut | mutation | SNP position | primer position |
|---|---|---|---|---|---|
| beta-101 | HBB g.-151C>T | wt | c | 0 | -161 >-141 |
| | | mut | t | | |
| beta-88c=a | HBB g.-138C>A | wt | c | 0 | -148>-128 |
| | | mut | a | | |
| beta-88c=t | HBB g.-138C>T | wt | c | 0 | -148>-128 |
| | | mut | t | | |
| beta-87c=a | HBB g.-137C>A | wt | c | 0 | -147>-127 |
| | | mut | a | | |
| beta-87c=t | HBB g.-137C>T | wt | c | 0 | -147>-127 |
| | | mut | t | | |
| beta-87c=g | HBB g.-137C>G | wt | c | 0 | -147>-127 |
| | | mut | g | | |
| beta-86 | HBB g.-136C>G | wt | c | 0 | -146>-126 |
| | | mut | g | | |
| beta-30 | HBB g.-80T>A | wt | t | 0 | -90>-70 |
| | | mut | a | | |
| beta-29 | HBB g.-79A>G | wt | a | 0 | -89>-69 |
| | | mut | g | | |
| beta-28a=g | HBB g.-78A>G | wt | a | 0 | -88>-68 |
| | | mut | g | | |
| beta-28a=c | HBB g.-78A>C | wt | a | 0 | -88>-68 |
| | | mut | c | | |
| betalnitCd | HBB g.2T>C | wt | t | 0 | -9>+12 |
| | | mut | c | | |
| betaCd5 | HBB g.17_18deICT | wt | ct | 0 | +7>+27 |
| | | mut | -ct | | +7>+28 |
| betaCd5+Cd2pol | HBB g.9C>T + g.17_18delCT | wt | c and ct | 0 | +7>+27 |
| | | mut | t and -ct | | +7>+28 |
| betaCd6-HbC | HBB g.19G>A | wt | g | 0 | +9>+29 |
| | | mut | a | | |
| betaCd6-HbS | HBB g.20A>T | wt | a | 0 | +10>+30 |
| | | mut | t | | |
| betaCd6=-a | HBB g.20delA | wt | a | 0 | +10>+30 |
| | | mut | -a | | |
| betaCd6-a+Cd2pol | HBB g.9C>T + g.20delA | wt | c and a | 0 | +10>+30 |
| | | mut | t and -a | | |
| betaCd8 | HBB g.25-26delAA | wt | aa | 0 | +16>+35 |
| | | mut | -aa | | +15>+36 |
| betaCd8+Cd2pol | HBB g.9C>T + g.25-26delAA | wt | c and aa | 0 | +16>+35 |
| | | mut | t and -aa | | +15>+36 |
| betaCd8-9 | HBB g.27_28insG | wt | g | 0 | +18>+37 |
| | | mut | gg | | |
| betaCd8-9+Cd2pol | HBB g.9C>T + g.27_28insG | wt | c and g | 0 | +18>+37 |
| | | mut | t and gg | | |
| betaCd9-10 | HBB g.30_31insT | wt | t | 0 | +21>+40 |
| | | mut | tt | | |
| betaCd14-15 | HBB g.45_46insG | wt | g | 0 | +36>+56 |
| | | mut | gg | | +36>+55 |
| betaCd15eerste | HBB g.46delT | wt | t | 0 | +36>+56 |
| | | mut | -t | | |
| betaCd15tweede | HBB g.47G>A | wt | g | 0 | +37>+57 |
| | | mut | a | | |
| betaCd15derde | HBB g.48G>A | wt | g | 0 | +38>+58 |
| | | mut | a | | |
| betaCd16 | HBB g.51delC | wt | c | 0 | +41>+61 |
| | | mut | -c | | |
| betaCd17 | HBB g.52A>T | wt | a | 0 | +42>+62 |
| | | mut | t | | |
| betaCd19 | HBB g.56A>G | wt | a | 0 | +46>+66 |
| | | mut | g | | |
| betaCd22 | HBB g.68_74delAAGTTGG | wt | aagttgg | 0 | +61 >+81 |
| | | mut | -aagttgg | | +58>+84 |
| betaCd26-HbE | HBB g.79G>A | wt | g | 0 | +69>+89 |
| | | mut | a | | |
| betaCd27-28 | HBB g.84_85insC | wt | c | 0 | +75>+95 |
| | | mut | cc | | +75>+94 |
| betaCd28-29 | HBB g.89delG | wt | g | 0 | +78>+98 |
| | | mut | -g | | |
| betaCd29 | HBB g.90C>T | wt | c | 0 | +80>+100 |
| | | mut | t | | |
| betaCd30 | HBB g.92G>C | wt | g | 0 | +82>+102 |
| | | mut | c | | |
| betalVS1-1g=t | HBB g.93G>T | wt | g | 0 | +83>+103 |
| | | mut | t | | |
| betalVS1-1g=a | HBB g.93G>A | wt | g | 0 | +83>+103 |
| | | mut | a | | |
| betalVS1-5g=c | HBB g.97G>C | wt | g | 0 | +87>+107 |
| | | mut | c | | |
| betaIVS1-5g=a | HBB g.97G>A | wt | g | 0 | +87>+107 |
| | | mut | a | | |
| betaIVS1-5g=t | HBB g.97G>T | wt | g | 0 | +87>+107 |
| | | mut | t | | |
| betalVS1-6 | HBB g.98T>C | wt | t | 0 | +88>+108 |
| | | mut | c | | |
| betalVS1del-25 | HBB g.202 to 226 del | wt | attttcccacccttaggctgctggt | 0 | +203>+223 |
| | | mut | -attttcccacccttaggctgctggt | | +203>+235 |
| betalVS1-110 | HBB g.202G>A | wt | g | 0 | +192>+212 |
| | | mut | a | | |
| betalVS1-128 | HBB g.220T>G | wt | t | 0 | +210>+230 |
| | | mut | g | | |
| betalVS1-130 | HBB g.222G>A | wt | g | 0 | +212>+232 |
| | | mut | a | | |
| betaCd35c=a | HBB g.238C>A | wt | c | 0 | +228>+248 |
| | | mut | a | | |
| betaCd35c=- | HBB g.238delC | wt | c | 0 | +228>+248 |
| | | mut | -c | | |
| betaCd36-37 | HBB g.240delT | wt | t | 0 | +231>+251 |
| | | mut | -t | | |
| betaCd39 | HBB g.248C>T | wt | c | 0 | +238>+258 |
| | | mut | t | | |
| betaCd41-42 | HBB g.254_257deITTCT | wt | ttct | 0 | +247>+266 |
| | | mut | -ttct | | +245>+268 |
| betaCd43 | HBB g.260G>T | wt | g | 0 | +250>+270 |
| | | mut | t | | |
| betaCd44 | HBB g.265delC | wt | c | 0 | +255>+275 |
| | | mut | -c | | |
| betaCd45 | HBB g.266delT | wt | t | 0 | +257>+277 |
| | | mut | -t | | |
| betaCd71-72 | HBBg.346_347insA | wt | a | 0 | +337>+356 |
| | | mut | aa | | |
| betaCd76 | HBB g.360delC | wt | c | 0 | +350>+370 |
| | | mut | -c | | |
| betaCd82-83 | HBB g.380delG | wt | g | 0 | +370>+390 |
| | | mut | -g | | |
| betalVS2-1 | HBB g.446G>A | wt | g | 0 | +436>+456 |
| | | mut | a | | |
| betaIVS2-1 +IVS2-16pol | HBB g.461C>G + g.446G>A | wt | c and g | 0 | +436>+456 |
| | | mut | g and a | | |
| betaDel-619 | HBB g.1015_1424+209del | wt | | 0 | +1025>+1043 |
| | | mut | -619bp | | +1005>+1014;+1634>+1643 |
| betaIVS2-654 | HBB g.1099C>T | wt | c | 0 | +1089>+1109 |
| | | mut | t | | |
| betalVS2-745 | HBB g.1190C>G | wt | c | 0 | +1180>+1200 |
| | | mut | g | | |
| betaIVS2-848 | HBB g.1293C>A | wt | c | 0 | +1283>+1303 |
| | | mut | a | | |
| betaCd121 | HBB g.1344G>T | wt | g | 0 | +1334>+1354 |
| | | mut | t | | |
| betaPolyA2 | HBB g.1424+20A>G | wt | a | 0 | +1525>+1545 |
| | | mut | g | | |
| betaPolyA1 | HBB g.1424+22A>G | wt | a | 0 | +1527>+1547 |
| | | mut | g | | |
| alfa1cd119 | HBA1 g.624C>T | wt | c | 0 | +614>+634 |
| | | mut | t | | |
| alfa1cd14nr1 | HBA1 g.43T>C | wt | t | 0 | +33>+53 |
| | | mut | c | | |
| alfa 1cd14nr2 | HBA1 g.44G>A | wt | g | 0 | +34>+54 |
| | | mut | a | | |
| alfa1cd14nr3 | HBA1 g.43T>A | wt | t | 0 | +33>+53 |
| | | mut | a | | |
| alfa2 initnr1 | HBA2g.1A>G | wt | a | 0 | -10>+11 |
| | | mut | g | | |
| alfa2 initnr2 | HBA2 g.2T>C | wt | t | 0 | -9>+12 |
| | | mut | c | | |
| alfa2 initnr3 | HBA2 g.2T>G | wt | t | 0 | -9>+12 |
| | | mut | g | | |
| alfa3.7(-2nt) | HBA1 g.-3_-2delAC | wt | ac | 0 | -12>+9 |
| | | mut | -ac | | -13>+9 |
| alfa2cd29 | HBA2 g.89T>C | wt | t | 0 | +79>+99 |
| | | mut | c | | |
| alfa2cd30 | HBA2 g.91_93delGAG | wt | gag | 0 | +82>+102 |
| | | mut | -gag | | +81>+103 |
| alfa 1cd30-31 | HBA1 g.94_95delAG | wt | ag | 0 | +85>+105 |
| | | mut | -ag | | +84>+105 |
| alfa 1cd31 | HBA1 g.96G>A | wt | g | 0 | +86>+106 |
| | | mut | a | | |
| alfa2lVS1(-5nt) | HBA2 g.97_101delTGAGG | wt | tgagg | 0 | +88>+108 |
| | | mut | -tgagg | | +86>+110 |
| alfa2lVS1-116 | HBA2g.211A>G | wt | a | 0 | +201>+221 |
| | | mut | g | | |
| alfa1lVS1-117 | HBA1 g.212G>A | wt | g | 0 | +202>+222 |
| | | mut | a | | |
| alfa1 51-55 | HBA1 g.272_284delGCTCTGCCCAGGT | wt | gctctgcccaggt | 0 | +268>+288 |
| | | mut | -gctctgcccaggt | | +262>+295 |
| alfa cd59 | HBA2 g.296G>A | wt | g | 0 | +286>+306 |
| | | mut | a | | |
| alfa2cd60-61 | HBA2 g.298_300delAAG | wt | aag | 0 | +292>+312 |
| | | mut | -aag | | +291>+313 |
| alfa 1cd62 | HBA1 g.304_306delGTG | wt | gtg | 0 | +295>+315 |
| | | mut | -gtg | | +294>+316 |
| alfa 1cd74-75 | HBA1 g.340_342delGAC | wt | gac | 0 | +331>+351 |
| | | mut | -gac | | +330>+352 |
| alfa21VS2-142 | HBA2 g.559G>A | wt | g | 0 | +549>+569 |
| | | mut | a | | |
| alfa 1lVS2-148 | HBA1 g.565A>G | wt | a | 0 | +555>+575 |
| | | mut | g | | |
| alfa2cd104 | HBA2 g.573G>A | wt | g | 0 | +563>+583 |
| | | mut | a | | |
| alfa2cd109 | HBA2 g.588T>G | wt | t | 0 | +578>+598 |
| | | mut | g | | |
| alfa cd110 | HBA2 g.591C>A | wt | c | 0 | +581>+601 |
| | | mut | a | | |
| alfa2cd116 | HBA2 g.608G>T | wt | g | 0 | +598>+618 |
| | | mut | t | | |
| alfa2cd112-116 | HBA2 g.598_609delCCTCCCCGCCGA | wt | cctccccgccga | 0 | +593>+613 |
| | | mut | -cctccccgccga | | +588>+619 |
| alfa2cd125(T-C) | HBA2 g.636T>C | wt | t | 0 | +626>+646 |
| | | mut | c | | |
| alfa2cd125(T-A) | HBA2 g.636T>A | wt | t | 0 | +626>+646 |
| | | mut | a | | |
| alfa2cd129 | HBA2 g.648T>C | wt | t | 0 | +638>+658 |
| | | mut | c | | |
| alfa cd130 | HBA2 g.650G>C | wt | g | 0 | +640>+660 |
| | | mut | c | | |
| alfa2cd131 | HBA2 g.653T>C | wt | t | 0 | +643>+663 |
| | | mut | c | | |
| alfa2cd142(T-C) | HBA2 g.686T>C | wt | t | 0 | +676>+696 |
| | | mut | c | | |
| alfa2cd142(T-A) | HBA2 g.686T>A | wt | t | 0 | +676>+696 |
| | | mut | a | | |
| alfa2cd142(T>G) | HBA2 g.686T>G | wt | t | 0 | +676>+696 |
| | | mut | g | | |
| alfa2cd142nr2 | HBA2 g.687A>C | wt | a | 0 | +677>+697 |
| | | mut | c | | |
| alfa2cd142nr3 | HBA2 g.688A>T | wt | a | 0 | +678>+698 |
| | | mut | t | | |
| alfa2(-16bp) | HBA2 g.688+74_688+89delCCTTCCTGGTCTTTGA | wt | ccttcctggtctttga | 0 | +759>+779 |
| | | mut | -ccttcctggtctttga | | +752>+787 |
| alfa2polyA1 | HBA2 g.688+94A>G | wt | a | 0 | +772>+792 |
| | | mut | g | | |
| alfa2polyA2 | HBA2 g.688+92A>G | wt | a | 0 | +770>+790 |
| | | mut | g | | |
| alfa2polyA3 | HBA2 g.688+93_688+94delAA | wt | aa | 0 | +771>+791 |
| | | mut | -aa | | +771>+792 |
| alfa2polyA4 | HBA2 g.688+94A>C | wt | a | 0 | +772>+792 |
| | | mut | c | | |

**Table 2 Experimental Design**

| PCR fragments in hyb. mixture | Exp. 1 | Exp. 2 | Exp. 3 | Exp. 4 | Exp. 5 | Exp. 6 |
|---|---|---|---|---|---|---|
| β FF+ER | Hom HBB g.20A>T | Het HBB g.20A>T and g.248C>T | Hom HBB g.27_28insG | Het HBB g.27_28insG and g.97G>C | Hom HBB g.248C>T | Hom HBB g.97G>C |
| β CF+QR | Wild type | Wild type | Het HBB g.1344G>T | Het HBB g.1424+20A>G | Het HBB g.1424+22A>G | Het HBB g.1190C>G |
| α₂ S13+S6 | Het HBA2 g.97_101delTGAGG and g.686T>C | Het HBA2 g.688+93_94delAA | Wild type | Wild type | Het HBA2 g.688+94A>G and g.688+92A>G | Wild type |
| α₁S13+S8 | Wild type | Wild type | Het HBA1 g.624C>T | Het HBA1 g.212G>A | Wild type | Wild type |

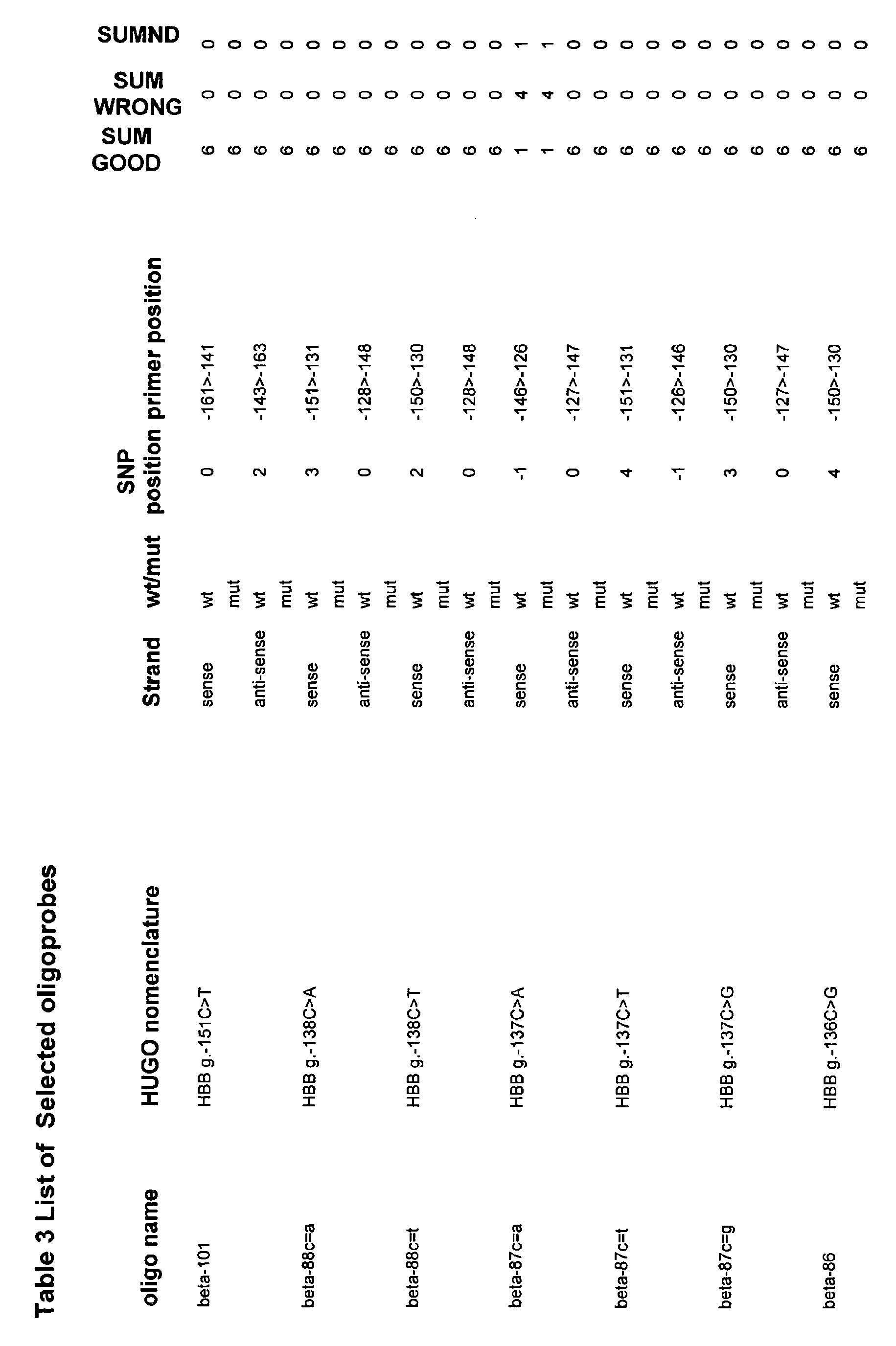

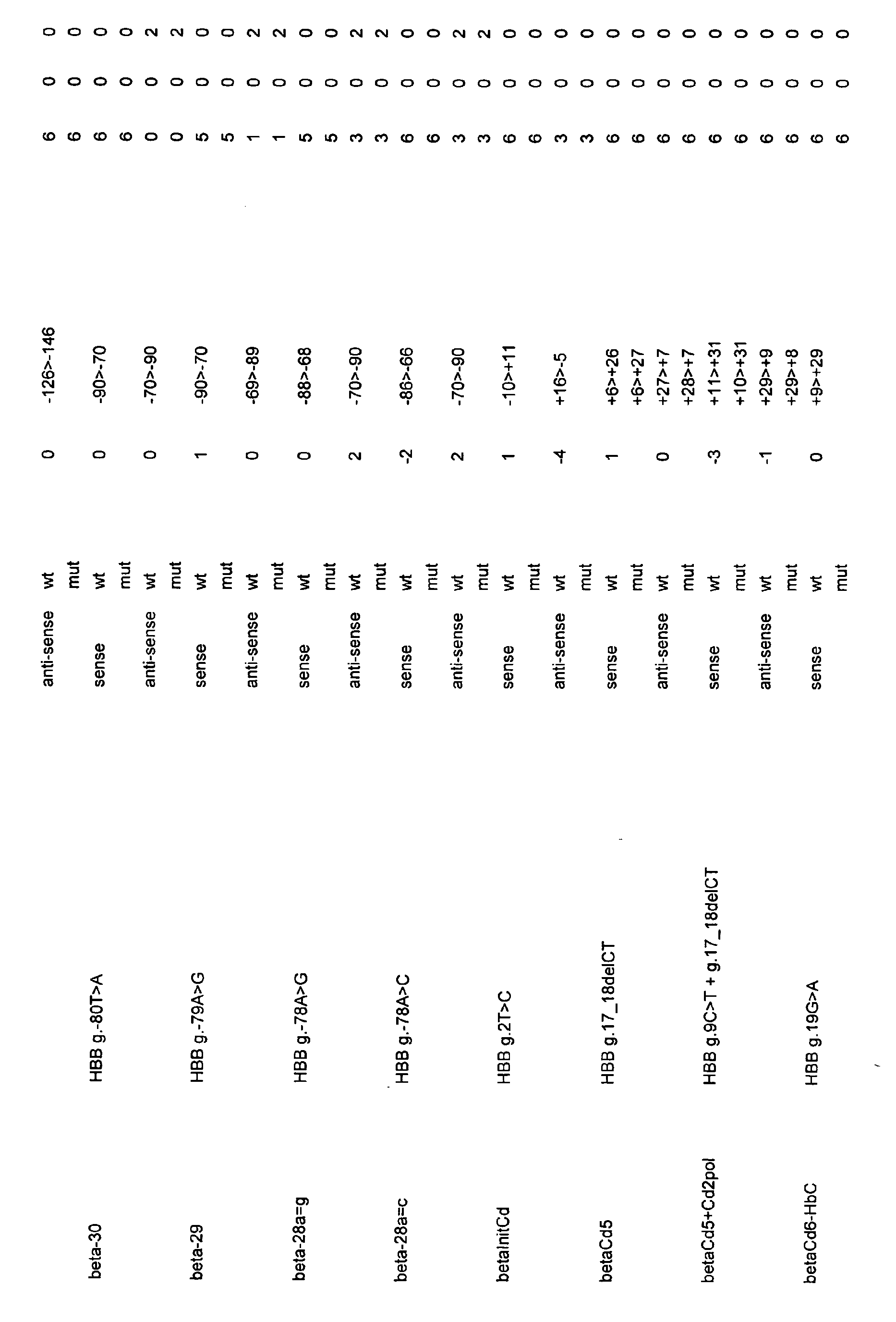

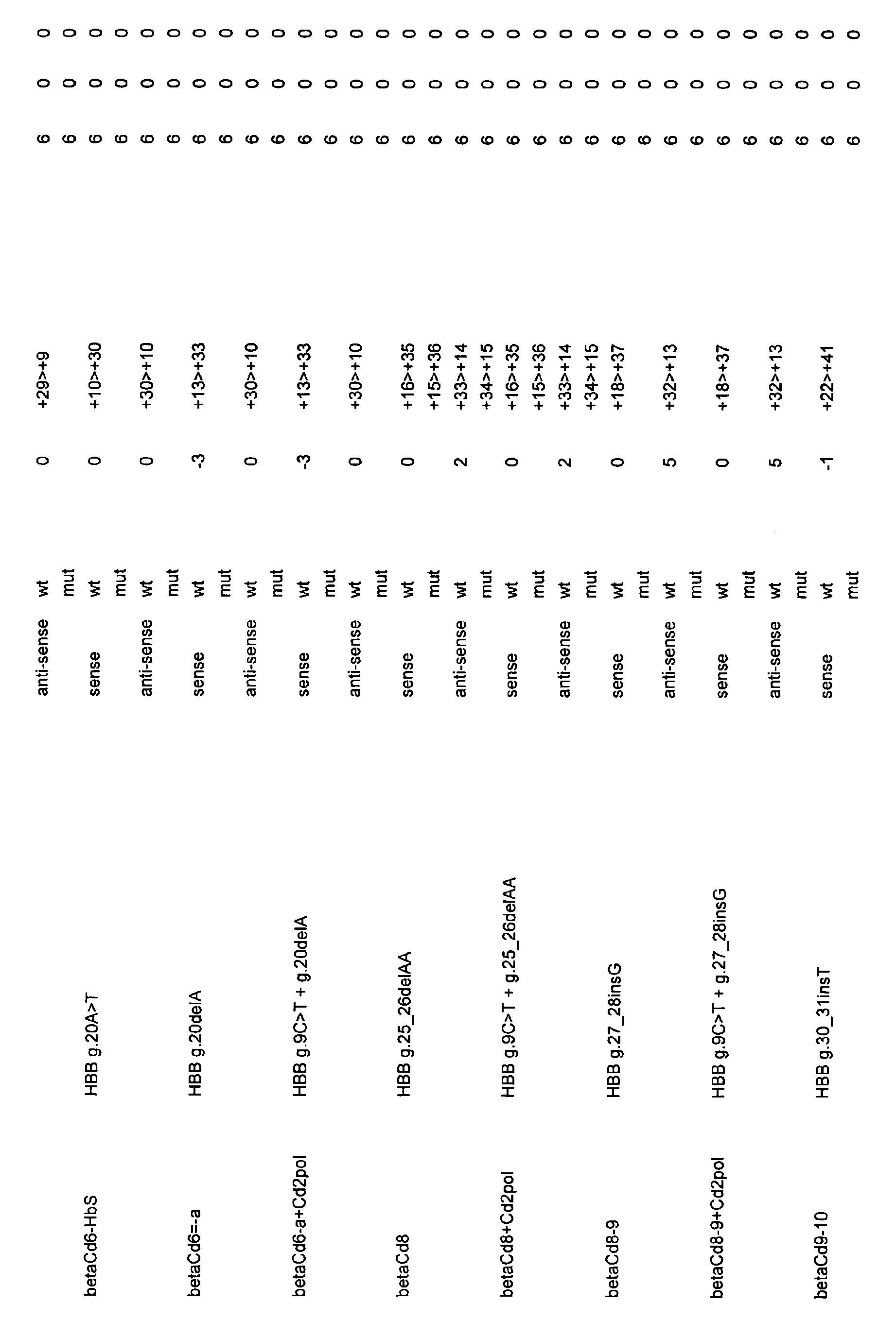

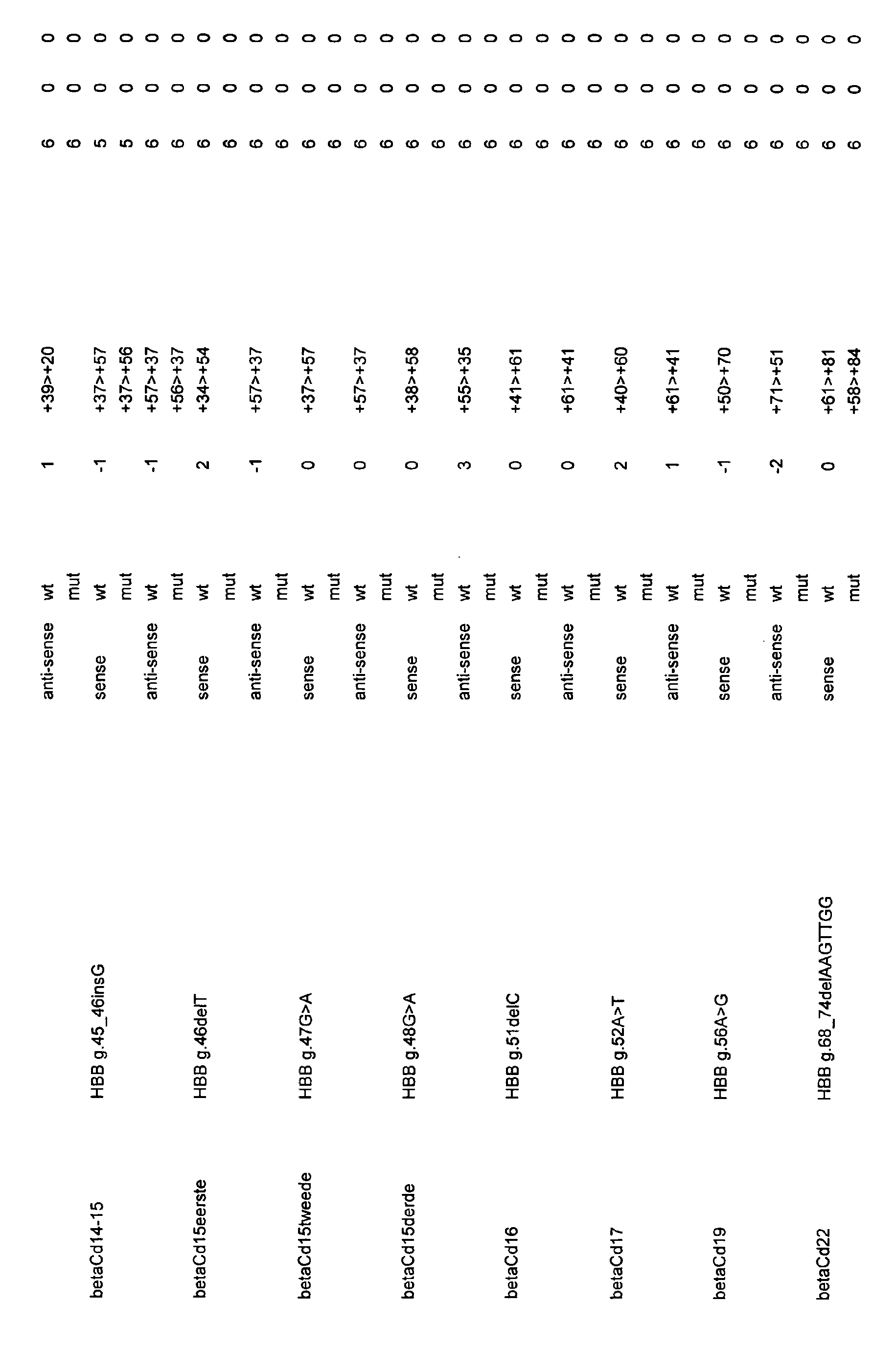

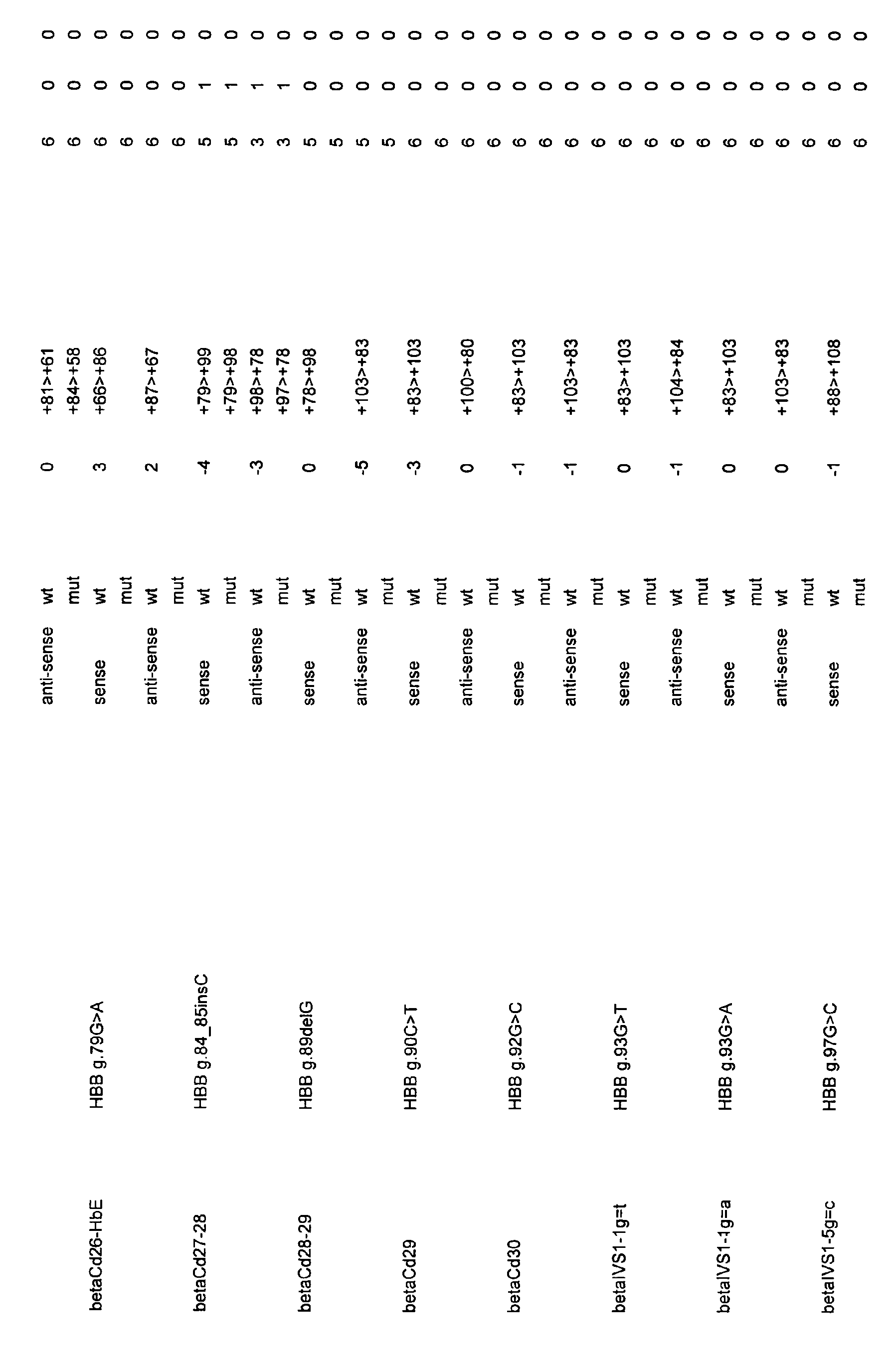

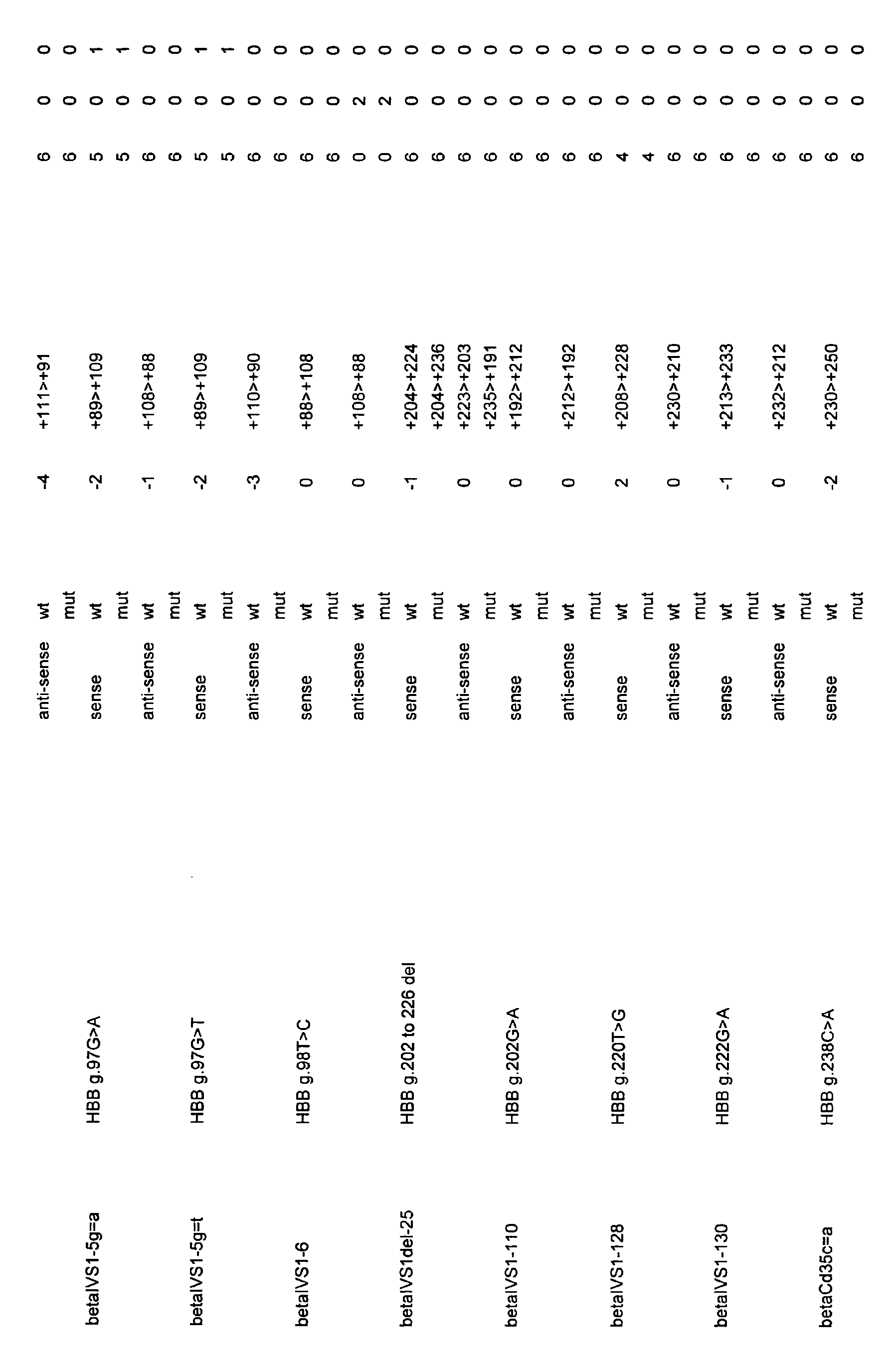

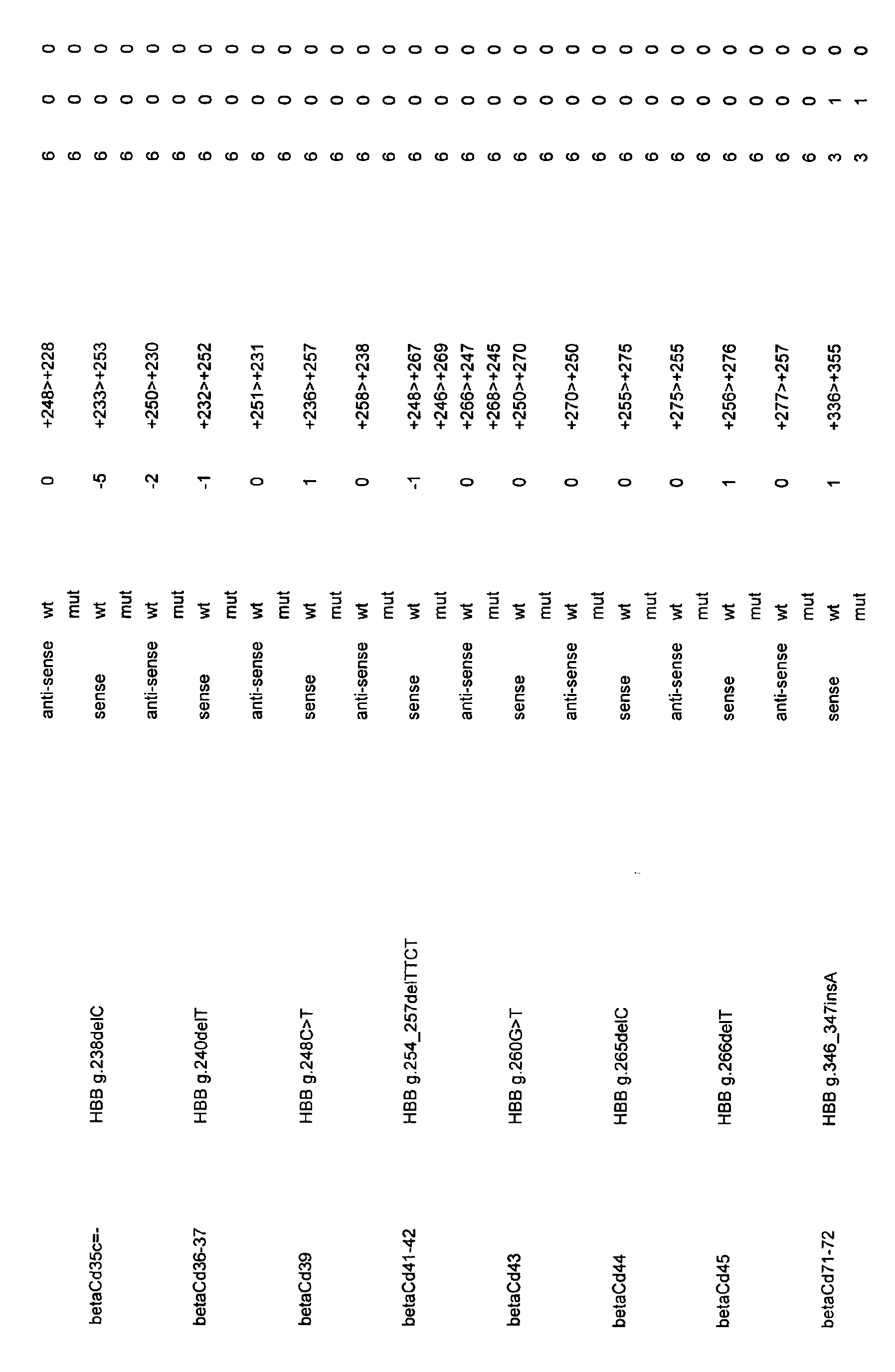

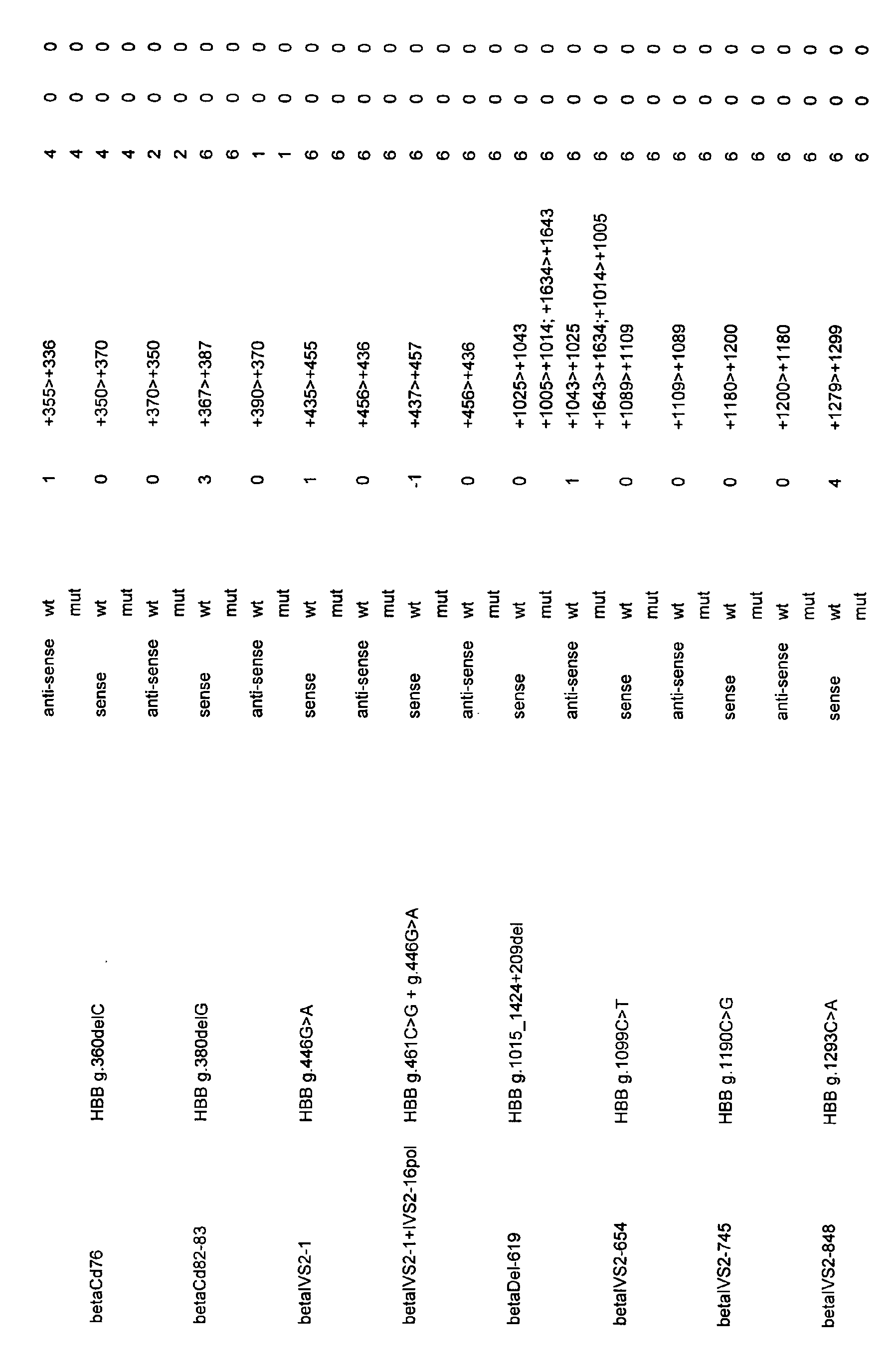

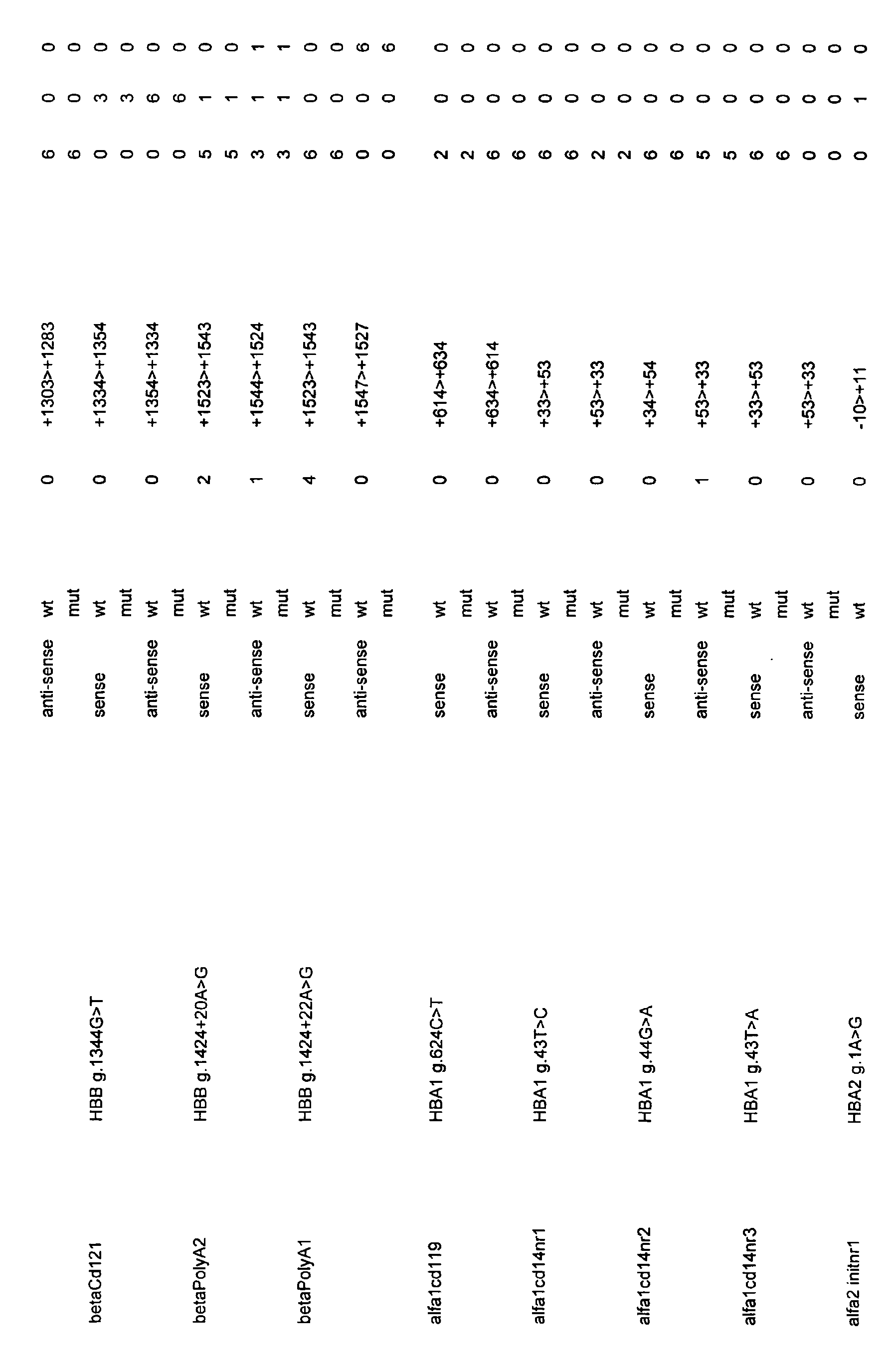

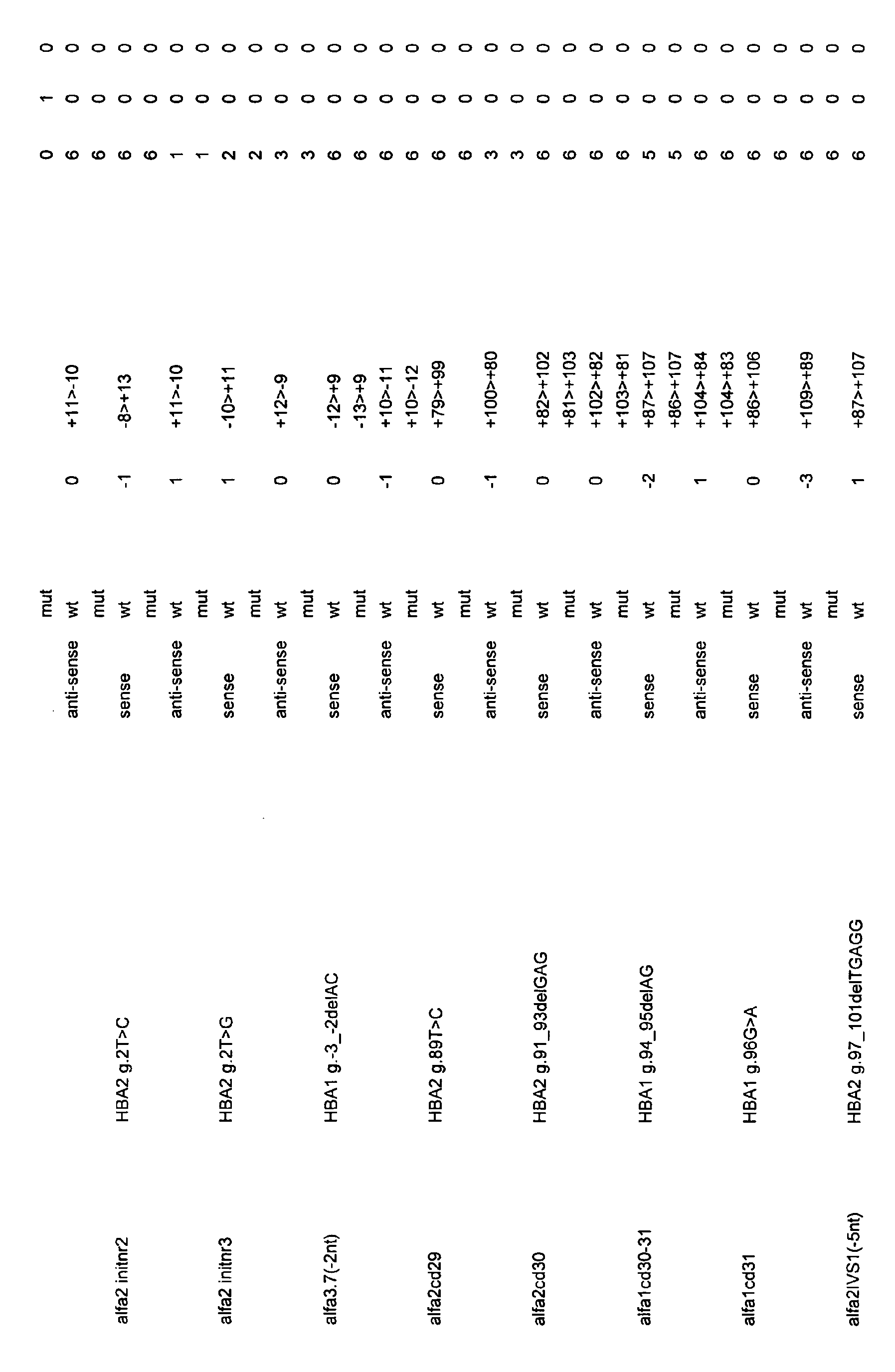

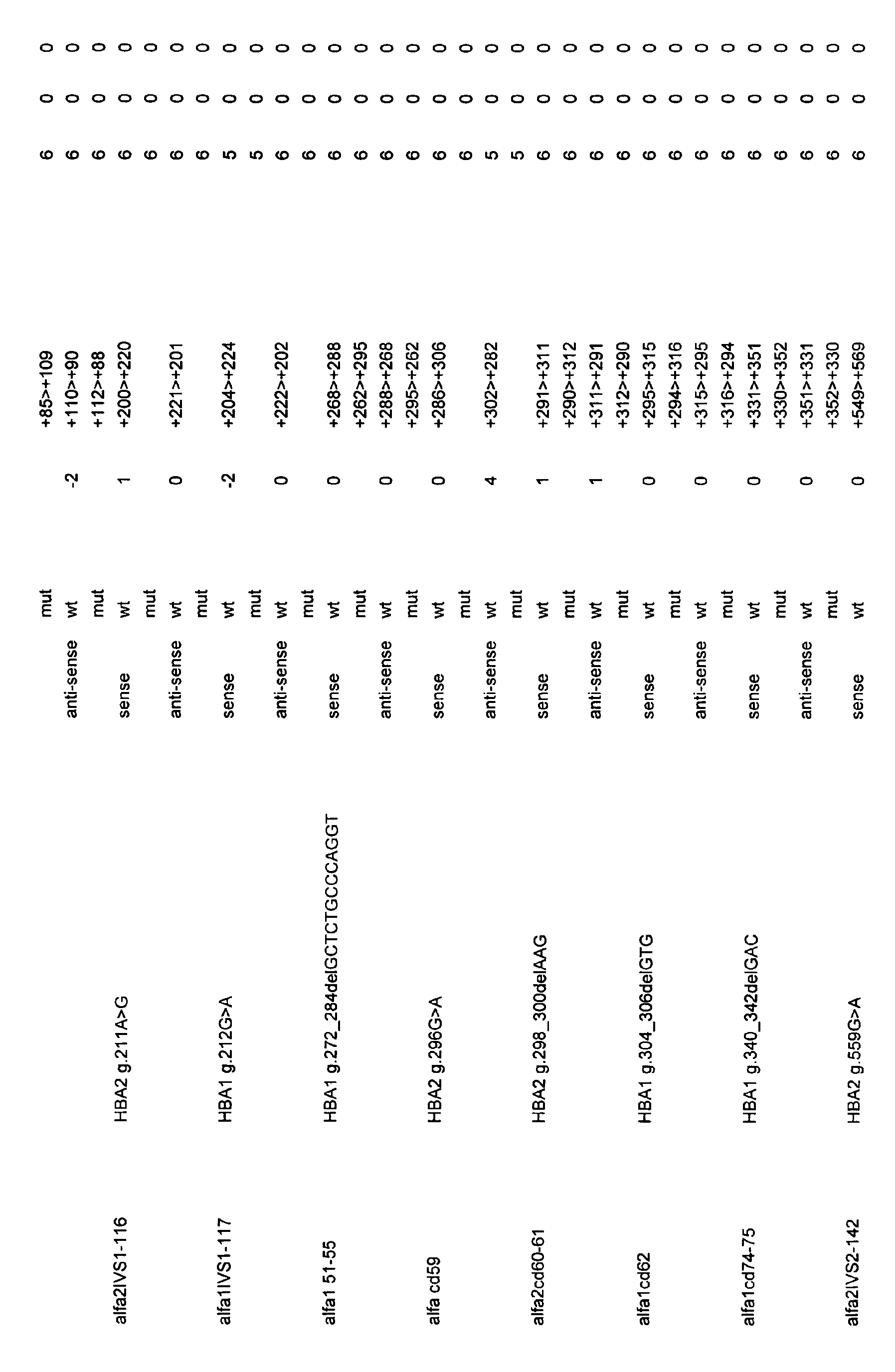

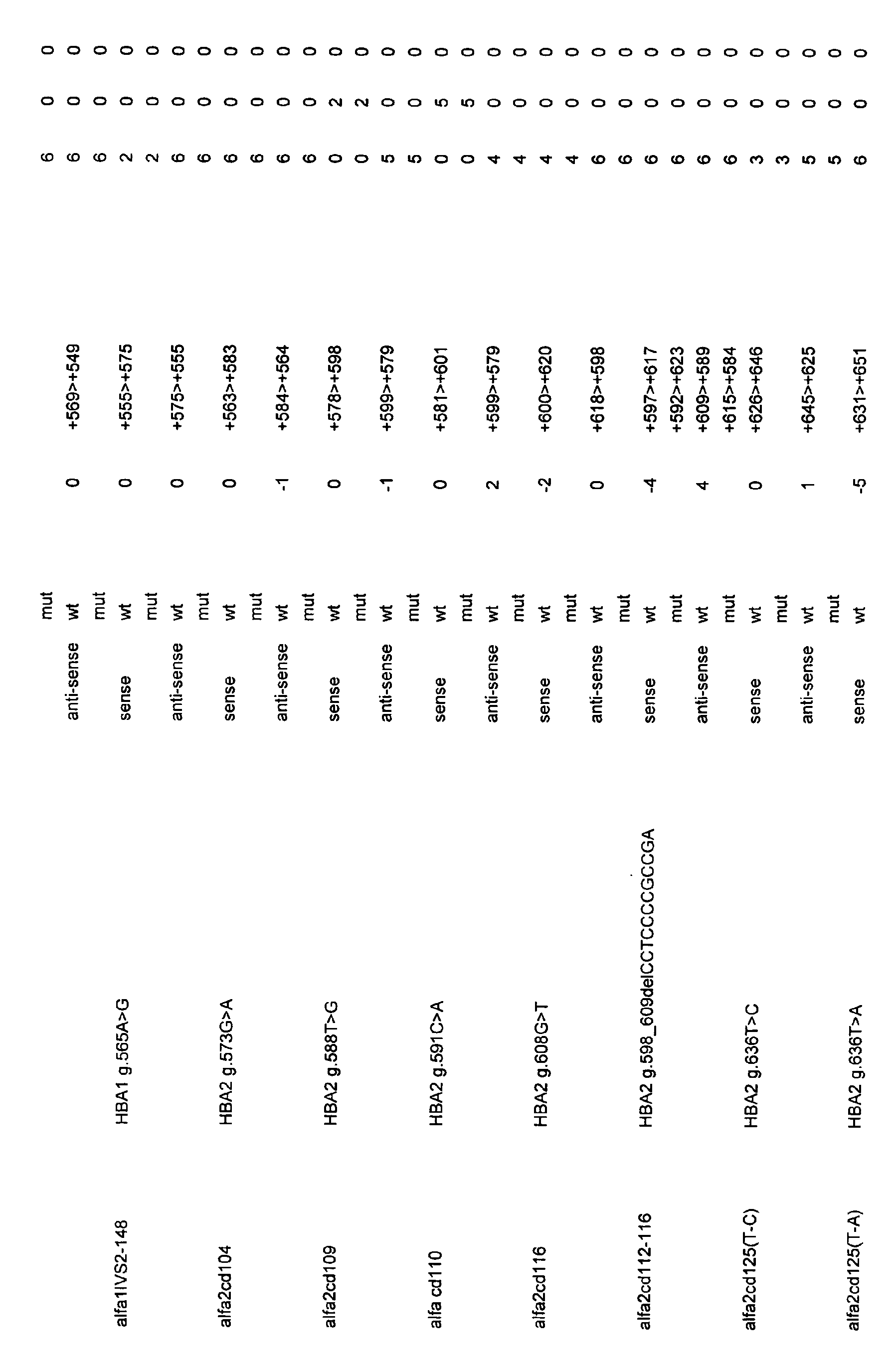

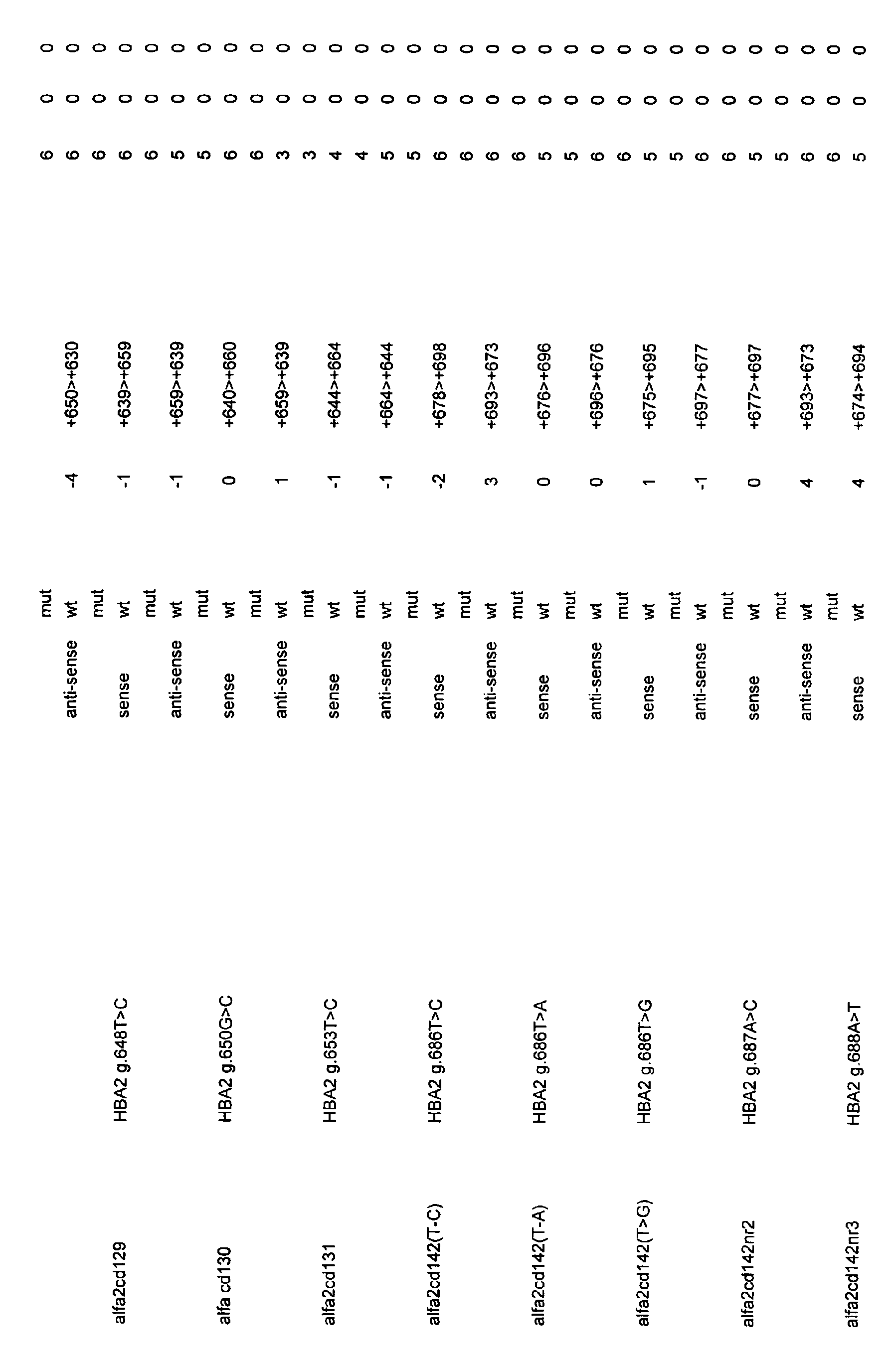

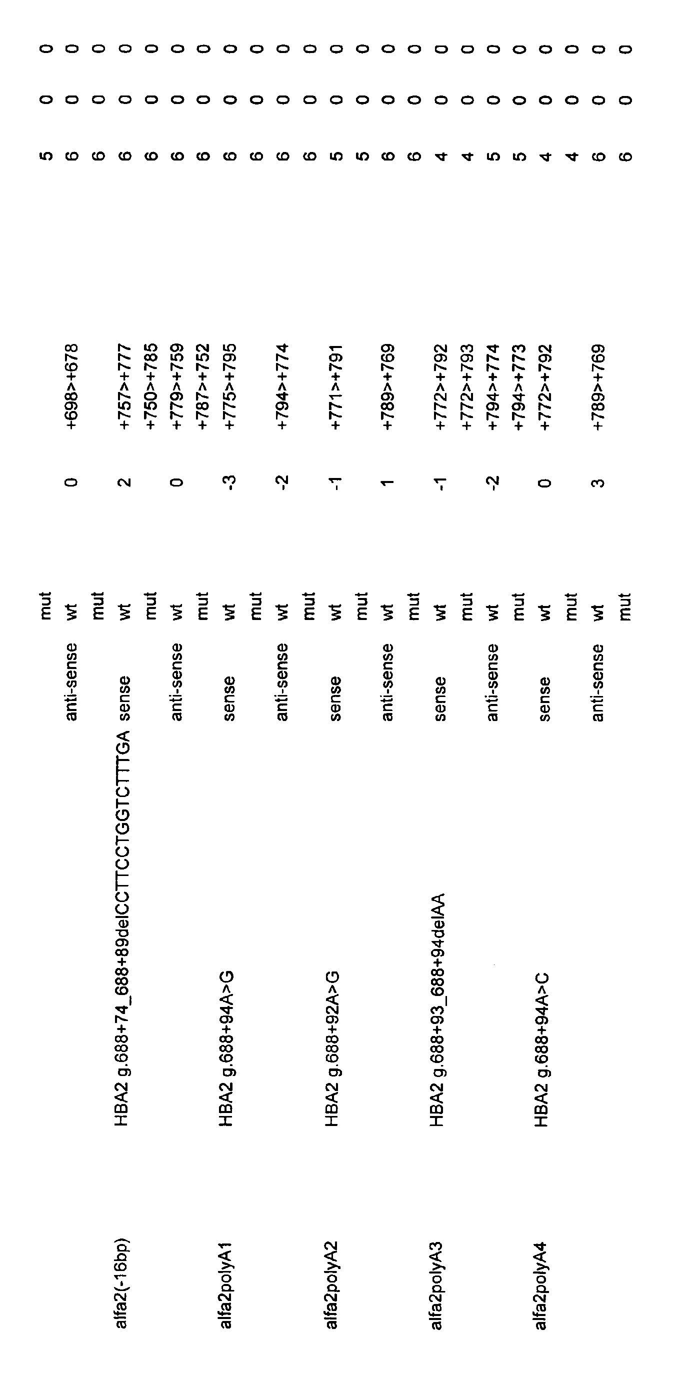

## Claims

1. A set of probes for detecting a mutant allele of an alpha- and/or beta-globin gene, said set comprising at least 10 different oligonucleotides having a continuous stretch of 20 and/or 21 nucleotides that comprise between zero and three mismatches compared to the mutant allele and wherein each of said oligonucleotides comprises a sequence specific for the mutation in the mutant allele and wherein each of said oligonucleotides comprises at one end of the continuous stretch at least 5 nucleotides of which at least 4 are specific for a wild type allele.

2. A set of probes according to claim 1, wherein each of said oligonucleotides comprises at either end at least 6 nucleotides of which at least 5 are specific for said wild type allele.

3. A set of probes according to any one of claims 1 or 2, wherein said set of probes comprises at least 11 21-mer oligonucleotides.

4. A set of probes according to any one of claims 1-3, wherein said mutant allele is an allele specified in table 1.

5. A set of probes according to any one of claims 1-4, wherein each of said oligonucleotides detects the same strand of the allele.

6. A collection of probes comprising at least 20 different sets of probes according to any one of claims 1-5.

7. A collection of probes according to claim 6, comprising sets of probes for at least 42 different alpha-globin mutant alleles specified in table 1.

8. A collection of probes according to any one of claims 6 or 7, comprising sets of probes for at least 66 different beta-globin mutant alleles specified in table 1.

9. A collection of probes according to any one of claims 6-8, comprising a set of probes for detecting a mutant allele and wherein each oligonucleotide in said set detects the same strand of the allele.

10. A collection of probes according to any one of claims 6-9, comprising two different sets of probes for detecting the same mutant allele and wherein each oligonucleotide in one set of probes detects one strand of the allele and wherein each oligonucleotide in the other set of probes detects the other strand of the allele.

11. A collection of probes according to any one of claims 6-10, wherein said collection further comprises a probe for detecting the corresponding wild type allele of at least one mutant allele.

12. A collection of probes, comprising at least one oligonucleotide of table 3 that is specific for an alpha-globin mutant allele or a beta-globin mutant allele.

13. A collection of probes according to claim 12, comprising at least 20 oligonucleotides of table 3 that are specific for an alpha-globin mutant allele or a beta-globin mutant allele.

14. A collection of probes according to any one of claims 12 or 13, comprising the 432 oligonucleotides specific for an alpha-globin mutant allele or a beta-globin mutant allele of table 3.

15. A collection of probes according to any one of claims 12-14, wherein said collection further comprises a probe for detecting the corresponding wild type allele of at least one mutant allele.

16. A collection of probes according to any one of claims 12-15, wherein at least one of said oligonucleotides is defined by a score of at least 1 for Sum Good in table 3.

17. A collection of probes according to any one of claims 12-16, wherein at least one of said oligonucleotides is defined by a score of 0 for Sum Wrong in table 3.

18. A collection of probes according to any one of claims 12-17, comprising probes for at least 42 different alpha-globin mutant alleles specified in table 3.

19. A collection of probes according to any one of claims 12-18, comprising probes for at least 66 different beta-globin mutant alleles specified in table 3.

20. A collection of probes according to any one of claims 12-19, comprising at least two different probes of table 3 for detecting the same mutant allele and wherein the oligonucleotide in one probe detects one strand of the allele and wherein the oligonucleotide in the other probe detects the other strand of the allele.

21. An array comprising at least one collection of probes according to any one of claims 6-20.

22. A kit of parts, comprising at least one collection of probes according to any one of claims 6-20 and a means of detection.

23. Use of a collection of probes according to any one of claim 6-20, and/or an array according to claim 21, and/or a kit of parts according to claim 22 for detecting mutations in alpha and/ or beta globin genes in a sample of DNA.

24. Use according to claim 23, for determining the presence of a hemoglobinopathy gene pattern in a sample of DNA.

25. Use according to any one of claims 23 or 24, for determining an increased risk of developing a blood related disorder.

26. A method of predicting an increased risk of developing an hemoglobinopathy in the progeny of a human individual comprising the steps of:
a) contacting a sample of DNA of said individual with a collection of probes according to any one of claims 6-20 and/or an array according to claim 21, and/or a kit of parts according to claim 22;
b) measuring binding of said sample of DNA to said collection of probes, and/or said array, and/or said kit of parts;
c) determining the presence of one or more mutations of an alpha or beta globin gene in said sample of DNA.

27. A method according to claim 26, which includes an amplification step.


### Annex to the application documents - subsequently filed sequences listing

SEQUENCE LISTING

<110>  Academisch Ziekenhuis Leiden

<120>  Disease specific ASO-probes for the detection of alpha- and
       beta-thalassemia mutations

<130>  P71011EP00

<140>  06075210.2
<141>  2006-02-01

<160>  117

<170>  PatentIn version 3.3

<210>  1
<211>  19
<212>  DNA
<213>  Artificial Sequence

<220>
<223>  Alpha2-globin gene specific reverse primer S6

<400>  1
tccattgttg gcacattcc                                                    19


<210>  2
<211>  20
<212>  DNA
<213>  Artificial Sequence

<220>
<223>  Alpha1-globin gene specific reverse primer S8

<400>  2
ctggcacgtt tctgagggaa                                                   20


<210>  3
<211>  42
<212>  DNA
<213>  Artificial Sequence

<220>
<223>  Forward primer M13S13

<400>  3
cgacgttgta aaacgacggc cagtcgccag ccaatgagcg cc                          42


<210>  4
<211>  21
<212>  DNA
<213>  Artificial Sequence

<220>
<223>  Primer beta FF

<400>  4
tgaagtccaa ctcctaagcc a                                                 21


<210>  5
<211>  20
<212>  DNA
<213>  Artificial Sequence

<220>
<223>  Primer beta-ER

<400>  5
aaacgatcct gagacttcca                                                    20


<210>  6
<211>  20
<212>  DNA
<213>  Artificial Sequence

<220>
<223>  Primer beta-CF

<400>  6
gtgtacacat attgaccaaa                                                    20


<210>  7
<211>  20
<212>  DNA
<213>  Artificial Sequence

<220>
<223>  Primer beta-Q

<400>  7
aaatgcactg acctcccaca                                                    20


<210>  8
<211>  25
<212>  DNA
<213>  Artificial Sequence

<220>
<223>  Deletion Oligoprobe betaIVS1del-25

<400>  8
attttcccac ccttaggctg ctggt                                              25


<210>  9
<211>  13
<212>  DNA
<213>  Artificial Sequence

<220>
<223>  Deletion Oligoprobe alfa1 51-55

<400>  9
gctctgccca ggt                                                           13


<210>  10
<211>  12
<212>  DNA
<213>  Artificial Sequence

<220>
<223>  Deletion Oligoprobe alfa2cd112-116

<400>  10
cctccccgcc ga                                                            12

<210>  11
<211>  16
<212>  DNA
<213>  Artificial Sequence

<220>
<223>  Deletion Oligoprobe alfa2(-16bp)

<400>  11
ccttcctggt ctttga                                                    16


<210>  12
<211>  21
<212>  DNA
<213>  Artificial Sequence

<220>
<223>  Fig. 1a - 21-mer oligonucleotide - wild-type probe

<400>  12
ctgactcctg aggagaagtc t                                              21


<210>  13
<211>  21
<212>  DNA
<213>  Artificial Sequence

<220>
<223>  Fig. 1a - 21-mer oligonucleotide - mutant probe

<400>  13
ctgactcctg aggtgaagtc t                                              21


<210>  14
<211>  21
<212>  DNA
<213>  Artificial Sequence

<220>
<223>  Fig. 1a - 21-mer oligonucleotide - mutant probe SNP position 5

<400>  14
tgcacctgac tcctgtggag a                                              21


<210>  15
<211>  21
<212>  DNA
<213>  Artificial Sequence

<220>
<223>  Fig. 1a - 21-mer oligonucleotide - mutant probe SNP position 4

<400>  15
gcacctgact cctgtggaga a                                              21


<210>  16
<211>  21
<212>  DNA
<213>  Artificial Sequence

<220>
<223>  Fig. 1a - 21-mer oligonucleotide - mutant probe SNP position 3

<400>  16
cacctgactc ctgtggagaa g                                                21

<210>  17
<211>  21
<212>  DNA
<213>  Artificial Sequence

<220>
<223>  Fig. 1a - 21-mer oligonucleotide - mutant probe SNP position 2

<400>  17
acctgactcc tgtggagaag t                                                21

<210>  18
<211>  21
<212>  DNA
<213>  Artificial Sequence

<220>
<223>  Fig. 1a - 21-mer oligonucleotide - mutant probe SNP position 1

<400>  18
cctgactcct gtggagaagt c                                                21

<210>  19
<211>  21
<212>  DNA
<213>  Artificial Sequence

<220>
<223>  Fig. 1a - 21-mer oligonucleotide - mutant probe SNP position -1

<400>  19
tgactcctga ggtgaagtct g                                                21

<210>  20
<211>  21
<212>  DNA
<213>  Artificial Sequence

<220>
<223>  Fig. 1a - 21-mer oligonucleotide - mutant probe SNP -2

<400>  20
gactcctgag gtgaagtctg c                                                21

<210>  21
<211>  21
<212>  DNA
<213>  Artificial Sequence

<220>
<223>  Fig. 1a - 21-mer oligonucleotide - mutant probe SNP position -3

<400>  21
actcctgagg tgaagtctgc c                                                21

<210>  22
<211>  21
<212>  DNA
<213>  Artificial Sequence

<220>
<223>  Fig. 1a - 21-mer oligonucleotide - mutant probe SNP position -4

<400>  22
ctcctgaggt gaagtctgcc g                                                21


<210>  23
<211>  21
<212>  DNA
<213>  Artificial Sequence

<220>
<223>  Fig. 1a - 21-mer oligonucleotide - mutant probe SNP position -5

<400>  23
tcctgaggtg aagtctgccg t                                                21


<210>  24
<211>  60
<212>  DNA
<213>  Artificial Sequence

<220>
<223>  Fig. 1b - probe design alfa2initnr1 - wt sequence sense

<400>  24
tggtccccac agactcagag agaacccacc atggtgctgt ctcctgccga caagaccaac      60


<210>  25
<211>  60
<212>  DNA
<213>  Artificial Sequence

<220>
<223>  FIg. 1b - probe design alfa2initnr1 - wt sequence anti-sense

<400>  25
gttggtcttg tcggcaggag acagcaccat ggtgggttct ctctgagtct gaggggacca      60


<210>  26
<211>  21
<212>  DNA
<213>  Artificial Sequence

<220>
<223>  Fig. 1b - probe design alfa2initnr1 - wt probe

<400>  26
agaacccacc atggtgctgt c                                                21


<210>  27
<211>  21
<212>  DNA
<213>  Artificial Sequence

<220>
<223>  Fig. 1b - probe design alfa2ininti1 - mut probe

<400>  27
agaacccacc gtggtgctgt c                                                21

<210>  28
<211>  21
<212>  DNA
<213>  Artificial Sequence

<220>
<223>  Fig. 1b - probe design alfa2initnr1 SNP position 5 wt sense

<400>  28
cagagagaac ccaccatggt g                                                21


<210>  29
<211>  21
<212>  DNA
<213>  Artificial Sequence

<220>
<223>  Fig. 1b - probe design alfa2initnr1 SNP position 4 wt sense

<400>  29
agagagaacc caccatggtg c                                                21


<210>  30
<211>  21
<212>  DNA
<213>  Artificial Sequence

<220>
<223>  Fig. 1b - probe design alfa2initnr1 SNP position 3 wt sense

<400>  30
gagagaaccc accatggtgc t                                                21


<210>  31
<211>  21
<212>  DNA
<213>  Artificial Sequence

<220>
<223>  Fig. 1b - probe design alfa2initnr1 SNP position 2 wt sense

<400>  31
agagaaccca ccgtggtgct g                                                21


<210>  32
<211>  21
<212>  DNA
<213>  Artificial Sequence

<220>
<223>  Fig. 1b - probe design alfa2initnr1 SNP position 1 wt sense

<400>  32
gagaacccac catggtgctg t                                                21


<210>  33
<211>  21
<212>  DNA
<213>  Artificial Sequence

<220>
<223>  Fig. 1b - probe design alfa2initnr1 SNP position -1 wt sense

<400>  33
gaacccacca tggtgctgtc t                                                21

<210>  34
<211>  21
<212>  DNA
<213>  Artificial Sequence

<220>
<223>  Fig. 1b - probe design alfa2initr1 SNP position -2 wt sense

<400>  34
aacccaccat ggtgctgtct c                                                21

<210>  35
<211>  21
<212>  DNA
<213>  Artificial Sequence

<220>
<223>  Fig. 1b - probe design alfa2initr1 SNP position -3 wt sense

<400>  35
acccaccatg gtgctgtctc c                                                21

<210>  36
<211>  21
<212>  DNA
<213>  Artificial Sequence

<220>
<223>  Fig. 1b - probe design alfa2initr1 SNP position -4 wt sense

<400>  36
cccaccatgg tgctgtctcc t                                                21

<210>  37
<211>  21
<212>  DNA
<213>  Artificial Sequence

<220>
<223>  Fig. 1b - probe design alfa2initr1 SNP position -5 wt sense

<400>  37
ccaccatggt gctgtctcct g                                                21

<210>  38
<211>  21
<212>  DNA
<213>  Artificial Sequence

<220>
<223>  Fig. 1b - probe design alfa2initr1 SNP position 5 mut sense

<400>  38
cagagagaac ccaccgtggt g                                                21

<210>  39
<211>  21
<212>  DNA
<213>  Artificial Sequence

<220>
<223>  Fig. 1b - probe design alfa2initnr1 SNP position 4 mut sense

<400>  39
agagagaacc caccgtggtg c                                                21


<210>  40
<211>  21
<212>  DNA
<213>  Artificial Sequence

<220>
<223>  Fig. 1b - probe design alfa2initnr1 SNP position 3 mut sense

<400>  40
gagagaaccc accgtggtgc t                                                21


<210>  41
<211>  21
<212>  DNA
<213>  Artificial Sequence

<220>
<223>  Fig. 1b - probe designa alfa2initr1 SNP position 2 mut sense

<400>  41
agagaaccca ccgtggtgct g                                                21


<210>  42
<211>  21
<212>  DNA
<213>  Artificial Sequence

<220>
<223>  Fig. 1b - probe design alfa2initnr1 SNP position 1 mut sense

<400>  42
gagaacccac cgtggtgctg t                                                21


<210>  43
<211>  21
<212>  DNA
<213>  Artificial Sequence

<220>
<223>  Fig.. 1b - probe design alfa2initnr1 SNP position -1 mut sense

<400>  43
gaacccaccg tggtgctgtc t                                                21


<210>  44
<211>  21
<212>  DNA
<213>  Artificial Sequence

<220>
<223>  Fig. 1b - probe design alfa2initnr1 SNP position -2 mut sense

<400>  44
aacccaccgt ggtgctgtct c                                                21

<210> 45
<211> 21
<212> DNA
<213> Artificial Sequence

<220>
<223> Fig. 1b - probe design alfa2initnr1 SNP position -3 mut sense

<400> 45
acccaccgtg gtgctgtctc c 21


<210> 46
<211> 21
<212> DNA
<213> Artificial Sequence

<220>
<223> Fig. 1b - probe design alfa2initnr1 SNP position -4 mut sense

<400> 46
cccaccgtgg tgctgtctcc t 21


<210> 47
<211> 21
<212> DNA
<213> Artificial Sequence

<220>
<223> Fig. 1b - probe design alfa2initr1 SNP position -5 mut sense

<400> 47
ccaccgtggt gctgtctcct g 21


<210> 48
<211> 21
<212> DNA
<213> Artificial Sequence

<220>
<223> Fig. 1b - probe design alfa2initnr1 SNP position 5 wt anti-sense

<400> 48
caccatggtg ggttctctct g 21


<210> 49
<211> 21
<212> DNA
<213> Artificial Sequence

<220>
<223> Fig. 1b - probe design alfa2initnr1 SNP position 4 wt anti-sense

<400> 49
gcaccatggt gggttctctc t 21


<210> 50
<211> 21
<212> DNA
<213> Artificial Sequence

<220>
<223> Fig. 1b - probe design alfa2initnr 1 SNP position 3 wt anti-sense

<400>  50
agcaccatgg tgggttctct c                                                21

<210>  51
<211>  21
<212>  DNA
<213>  Artificial Sequence

<220>
<223>  Fig. 1b - probe design alfa2initnr1 SNP position 2 wt anti-sense

<400>  51
cagcaccatg gtgggttctc t                                                21

<210>  52
<211>  21
<212>  DNA
<213>  Artificial Sequence

<220>
<223>  Fig. 1b - probe design alfa2initnr1 SNP position 1 wt anti-sense

<400>  52
acagcaccat ggtgggttct c                                                21

<210>  53
<211>  21
<212>  DNA
<213>  Artificial Sequence

<220>
<223>  Fig. 1b - probe design alfa2initnr1 SNP position -1 wt anti-sense

<400>  53
agacagcacc atggtgggtt c                                                21

<210>  54
<211>  21
<212>  DNA
<213>  Artificial Sequence

<220>
<223>  Fig. 1 - probe design alfa2initnr1 SNP position -2 wt anti-sense

<400>  54
gagacagcac catggtgggt t                                                21

<210>  55
<211>  21
<212>  DNA
<213>  Artificial Sequence

<220>
<223>  Fig. 1b - probe design alfa2initnr1 SNP position -3 wt anti-sense

<400>  55
ggagacagca ccatggtggg t                                                21

<210>  56
<211>  21
<212>  DNA
<213>  Artificial Sequence

<220>
<223>  Fig. 1b - probe design alfa2initnr1 SNP position -4 wt anti-sense

<400>  56
aggagacagc accatggtgg g                                                21


<210>  57
<211>  21
<212>  DNA
<213>  Artificial Sequence

<220>
<223>  Fig. 1b - probe design alfa2initnr1 SNP position -5 mut
       anti-sense

<400>  57
caggagacag caccatggtg g                                                21


<210>  58
<211>  21
<212>  DNA
<213>  Artificial Sequence

<220>
<223>  Fig. 1b - probe design alfa2initnr1 SNP position 5 mut anti-sense

<400>  58
caccacggtg ggttctctct g                                                21


<210>  59
<211>  21
<212>  DNA
<213>  Artificial Sequence

<220>
<223>  Fig. 1b - probe design alfa2initnr1 SNP position 4 mut anti-sense

<400>  59
gcaccacggt gggttctctc t                                                21


<210>  60
<211>  21
<212>  DNA
<213>  Artificial Sequence

<220>
<223>  Fig. 1b - probe design alfa2initnr1 SNP position 3 mut anti-sense

<400>  60
agcaccacgg tgggttctct c                                                21


<210>  61
<211>  21
<212>  DNA
<213>  Artificial Sequence

<220>
<223>  Fig. 1b - probe design alfa2initnr1 SNP position 2 mut anti-sense

<400>  61
cagcaccacg gtgggttctc t                                                21

<210>  62
<211>  21
<212>  DNA
<213>  Artificial Sequence

<220>
<223>  Fig. 1b - probe design alfa2initnr1 SNP position 1 mut anti-sense

<400>  62
acagcaccac ggtgggttct c                                                21


<210>  63
<211>  21
<212>  DNA
<213>  Artificial Sequence

<220>
<223>  Fig. 1b - probe design alfa2initnr1 SNP position -1 mut
       anti-sense

<400>  63
agacagcacc acggtgggtt c                                                21


<210>  64
<211>  21
<212>  DNA
<213>  Artificial Sequence

<220>
<223>  Fig. 1b - probe design alfa2initnr1 SNP position -2 mut
       anti-sense

<400>  64
gagacagcac cacggtgggt t                                                21


<210>  65
<211>  21
<212>  DNA
<213>  Artificial Sequence

<220>
<223>  Fig. 1b - probe design alfa2initnr1 SNP position -3 mut
       anti-sense

<400>  65
ggagacagca ccacggtggg t                                                21


<210>  66
<211>  21
<212>  DNA
<213>  Artificial Sequence

<220>
<223>  Fig. 1b - probe design alfa2initnr1 SNP position -4 mut
       anti-sense

<400>  66
aggagacagc accacggtgg g                                                21


<210>  67
<211>  21
<212>  DNA

<213>  Artificial Sequence

<220>
<223>  Fig. 1b - probe design alfa2initnr1 SNP position -5 mut
      anti-sense

<400>  67
caggagacag caccacggtg g                                                21


<210>  68
<211>  40
<212>  DNA
<213>  Artificial Sequence

<220>
<223>  Beta Cd5 - wt sequence - sense

<400>  68
atggtgcacc tgactcctga ggagaagtct gccgttactg                            40


<210>  69
<211>  40
<212>  DNA
<213>  Artificial Sequence

<220>
<223>  Beta Cd5 - wt sequence anti-sense

<400>  69
cagtaacggc agacttctcc tcaggagtca ggtgcaccat                            40


<210>  70
<211>  21
<212>  DNA
<213>  Artificial Sequence

<220>
<223>  BetaCd5 - wt probe sense

<400>  70
cacctgactc ctgaggagaa g                                                21


<210>  71
<211>  20
<212>  DNA
<213>  Artificial Sequence

<220>
<223>  BetaCd5 - mut probe sense

<400>  71
cacctgactc gaggagaagt                                                  20


<210>  72
<211>  21
<212>  DNA
<213>  Artificial Sequence

<220>
<223>  BetaCd5 - wt sense SNP 5

<400>  72
tggtgcacct gactcctgag g                                                21

<210>  73
<211>  21
<212>  DNA
<213>  Artificial Sequence

<220>
<223>  BetaCd5 - wt sense SNP 4

<400>  73
ggtgcacctg actcctgagg a                                                21


<210>  74
<211>  21
<212>  DNA
<213>  Artificial Sequence

<220>
<223>  BetaCd5 - wt sense SNP 3

<400>  74
gtgcacctga ctcctgagga g                                                21


<210>  75
<211>  21
<212>  DNA
<213>  Artificial Sequence

<220>
<223>  Beta Cd5 - wt sense SNP 2

<400>  75
tgcacctgac tcctgaggag a                                                21


<210>  76
<211>  21
<212>  DNA
<213>  Artificial Sequence

<220>
<223>  Beta Cd5 - wt sense SNP 1

<400>  76
gcacctgact cctgaggaga a                                                21


<210>  77
<211>  21
<212>  DNA
<213>  Artificial Sequence

<220>
<223>  Beta Cd5 - wt sense SNP -1

<400>  77
acctgactcc tgaggagaag t                                                21


<210>  78
<211>  21
<212>  DNA
<213>  Artificial Sequence

<220>

<223>  Beta Cd5 - wt sense SNP -2

<400>  78
cctgactcct gaggagaagt c                                                21


<210>  79
<211>  21
<212>  DNA
<213>  Artificial Sequence

<220>
<223>  Beta Cd5 - wt sense SNP -3

<400>  79
ctgactcctg aggagaagtc t                                                21


<210>  80
<211>  21
<212>  DNA
<213>  Artificial Sequence

<220>
<223>  Beta Cd5 - wt sense SNP -4

<400>  80
tgactcctga ggagaagtct g                                                21


<210>  81
<211>  21
<212>  DNA
<213>  Artificial Sequence

<220>
<223>  BetaCd5 - wt sense SNP -5

<400>  81
gactcctgag gagaagtctg c                                                21


<210>  82
<211>  20
<212>  DNA
<213>  Artificial Sequence

<220>
<223>  BetaCd5 - mut sense SNP 5

<400>  82
tggtgcacct gactcgagga                                                  20


<210>  83
<211>  20
<212>  DNA
<213>  Artificial Sequence

<220>
<223>  Beta Cd5 - mut sense SNP 4

<400>  83
ggtgcacctg actcgaggag                                                  20


<210>  84
<211>  20

<212>  DNA
<213>  Artificial Sequence

<220>
<223>  BetaCd5 - mut sense SNP 3

<400>  84
gtgcacctga ctcgaggaga                                                    20


<210>  85
<211>  20
<212>  DNA
<213>  Artificial Sequence

<220>
<223>  BetaCd5 - mut sense SNP 2

<400>  85
tgcacctgac tcgaggagaa                                                    20


<210>  86
<211>  20
<212>  DNA
<213>  Artificial Sequence

<220>
<223>  BetaCD5 - mut sense SNP 1

<400>  86
gcacctgact cgaggagaag                                                    20


<210>  87
<211>  20
<212>  DNA
<213>  Artificial Sequence

<220>
<223>  BetaCd5 - mut sense SNP -1

<400>  87
acctgactcg aggagaagtc                                                    20


<210>  88
<211>  20
<212>  DNA
<213>  Artificial Sequence

<220>
<223>  BetaCd5 - mut sense SNP -2

<400>  88
cctgactcga ggagaagtct                                                    20


<210>  89
<211>  20
<212>  DNA
<213>  Artificial Sequence

<220>
<223>  BetaCd5 - mut sense SNP -3

<400>  89
ctgactcgag gagaagtctg                                                    20

<210>  90
<211>  20
<212>  DNA
<213>  Artificial Sequence

<220>
<223>  BetaCd5 - mut sense SNP -4

<400>  90
tgactcgagg agaagtctgc                                                    20


<210>  91
<211>  20
<212>  DNA
<213>  Artificial Sequence

<220>
<223>  BetaCD5 - mut sense SNP -5

<400>  91
gactcgagga gaagtctgcc                                                    20


<210>  92
<211>  21
<212>  DNA
<213>  Artificial Seqence

<220>
<223>  BetaCd5 - wt anti-sense SNP 5

<400>  92
cctcaggagt caggtgcacc a                                                  21


<210>  93
<211>  21
<212>  DNA
<213>  Artificial Sequence

<220>
<223>  BetaCd5 - wt anti-sense SNP 4

<400>  93
tcctcaggag tcaggtgcac c                                                  21


<210>  94
<211>  21
<212>  DNA
<213>  Artificial Sequence

<220>
<223>  BetaCD5 - wt anti-sense SNP 3

<400>  94
ctcctcagga gtcaggtgca c                                                  21


<210>  95
<211>  21
<212>  DNA
<213>  Artificial Sequence

<220>

<223>  BetaCd5 - wt anti-sense SNP 2

<400>  95
tctcctcagg agtcaggtgc a                                                21


<210>  96
<211>  21
<212>  DNA
<213>  Artificial Sequence

<220>
<223>  BetaCd5 - wt anti-sense SNP 1

<400>  96
ttctcctcag gagtcaggtg c                                                21


<210>  97
<211>  21
<212>  DNA
<213>  Artificial Sequence

<220>
<223>  BetaCd5 - wt anti-sense SNP -1

<400>  97
acttctcctc aggagtcagg t                                                21


<210>  98
<211>  21
<212>  DNA
<213>  Artificial Sequence

<220>
<223>  BetaCd5 - wt anti-sense SNP -2

<400>  98
gacttctcct caggagtcag g                                                21


<210>  99
<211>  21
<212>  DNA
<213>  Artificial Sequence

<220>
<223>  BetaCd5 - wt anti-sense SNP -3

<400>  99
agacttctcc tcaggagtca g                                                21


<210>  100
<211>  21
<212>  DNA
<213>  Artificial Sequence

<220>
<223>  BetaCd5 - wt anti-sense SNP -4

<400>  100
cagacttctc ctcaggagtc a                                                21


<210>  101
<211>  21

<212>  DNA
<213>  Artificial Sequence

<220>
<223>  BetaCd5 - wt anti-sense SNP -5

<400>  101
gcagacttct cctcaggagt c                                                21


<210>  102
<211>  20
<212>  DNA
<213>  Artificial Sequence

<220>
<223>  BetaCd5 - mut anti-sense SNP 5

<400>  102
tcctcgagtc aggtgcacca                                                  20


<210>  103
<211>  20
<212>  DNA
<213>  Artificial Sequence

<220>
<223>  BetaCd5 - mut anti-sense SNP 4

<400>  103
ctcctcgagt caggtgcacc                                                  20


<210>  104
<211>  20
<212>  DNA
<213>  Artificial Sequence

<220>
<223>  BetaCd5 - mut anti-sense SNP 3

<400>  104
tctcctcgag tcaggtgcac                                                  20


<210>  105
<211>  20
<212>  DNA
<213>  Artificial Sequence

<220>
<223>  BetaCd5 - mut anti-sense SNP 2

<400>  105
ttctcctcga gtcaggtgca                                                  20


<210>  106
<211>  20
<212>  DNA
<213>  Artificial Sequence

<220>
<223>  BetaCd5 - mut anti-sense SNP 1

<400>  106
cttctcctcg agtcaggtgc                                                  20

<210>  107
<211>  20
<212>  DNA
<213>  Artificial Sequence

<220>
<223>  BetaCd5 - mut anti-sense SNP -1

<400>  107
gacttctcct cgagtcaggt                                                    20


<210>  108
<211>  20
<212>  DNA
<213>  Artificial Sequence

<220>
<223>  BetaCd5 - mut anti-sense SNP -2

<400>  108
agacttctcc tcgagtcagg                                                    20


<210>  109
<211>  20
<212>  DNA
<213>  Artificial Sequence

<220>
<223>  BetaCd5 - mut anti-sense SNP -3

<400>  109
cagacttctc ctcgagtcag                                                    20


<210>  110
<211>  20
<212>  DNA
<213>  Artificial Sequence

<220>
<223>  BetaCd5 - mut anti-sense SNP -4

<400>  110
gcagacttct cctcgagtca                                                    20


<210>  111
<211>  20
<212>  DNA
<213>  Artificial Sequence

<220>
<223>  BetaCd5 - mut anti-sense SNP -5

<400>  111
ggcagacttc tcctcgagtc                                                    20


<210>  112
<211>  1131
<212>  DNA
<213>  Homo sapiens alpha-2 globin

<220>
<221>  CDS
<222>  (338)..(432)

<220>
<221>  CDS
<222>  (550)..(754)

<220>
<221>  CDS
<222>  (897)..(1022)

<400>  112
cacgagccga cagcgcccga ccccaacggg ccggccccgc cagcgccgct accgccctgc      60
ccccgggcga gcgggatggg cgggagtgga gtggcgggtg gagggtggag acgtcctggc     120
ccccgccccg cgtgcacccc caggggaggc cgagcccgcc gcccggcccc gcgcaggccc     180
cgcccgggac tcccctgcgg tccaggccgc gccccgggct ccgcgccagc caatgagcgc     240
cgcccggccg ggcgtgcccc cgcgccccaa gcataaaccc tggcgcgctc gcgggccggc     300
actcttctgg tccccacaga ctcagagaga acccacc atg gtg ctg tct cct gcc     355
gac aag acc aac gtc aag gcc gcc tgg ggt aag gtc ggc gcg cac gct     403
ggc gag tat ggt gcg gag gcc ctg gag ag  gtgaggctcc ctcccctgct       452
ccgacccggg ctcctcgccc gcccggaccc acaggccacc ctcaaccgtc ctggccccgg     512
acccaaaccc cacccctcac tctgcttctc cccgcag g atg ttc ctg tcc ttc       565
ccc acc acc aag acc tac ttc ccg cac ttc gac ctg agc cac ggc tct     613
gcc cag gtt aag ggc cac ggc aag aag gtg gcc gac gcg ctg acc aac     661
gcc gtg gcg cac gtg gac gac atg ccc aac gcg ctg tcc gcc ctg agc     709
gac ctg cac gcg cac aag ctt cgg gtg gac ccg gtc aac ttc aag         754
gtgagcggcg ggccgggagc gatctgggtc gaggggcgag atggcgcctt cctctcaggg     814
cagaggatca cgcgggttgc gggaggtgta gcgcaggcgg cggctgcggg cctgggccgc     874
actgaccctc ttctctgcac ag ctc cta agc cac tgc ctg ctg gtg acc ctg     926
gcc gcc cac ctc ccc gcc gag ttc acc cct gcg gtg cac gcc tcc ctg     974
gac aag ttc ctg gct tct gtg agc acc gtg ctg acc tcc aaa tac cgt     1022
taagctggag cctcggtagc cgttcctcct gcccgctggg cctcccaacg ggccctcctc     1082
ccctccttgc accggccctt cctggtcttt gaataaagtc tgagtgggc                 1131

<210>  113
<211>  142
<212>  PRT
<213>  Homo sapiens alpha-2 globin

<400>  113

<210>  114
<211>  1442
<212>  DNA
<213>  Homo sapiens alpha1-globin

<220>
<221>  CDS
<222>  (338)..(432)

<220>
<221>  CDS
<222>  (550)..(754)

<220>

<221>  CDS
<222>  (904)..(1029)

<400>  114
cacgagccga cagcgcccga ccccaacggg ccggccccgc cagcgccgct accgccctgc      60
ccccgggcga gcgggatggg cgggagtgga gtggcgggtg gagggtggag acgtcctggc     120
ccccgccccg cgtgcacccc caggggaggc cgagcccgcc gcccggcccc gcgcaggccc     180
cgcccgggac tcccctgcgg tccaggccgc gccccgggct ccgcgccagc caatgagcgc     240
cgcccggccg ggcgtgcccc cgcgccccaa gcataaaccc tggcgcgctc gcggcccggc     300
actcttctgg tccccacaga ctcagagaga acccacc atg gtg ctg tct cct gcc      355
gac aag acc aac gtc aag gcc gcc tgg ggt aag gtc ggc gcg cac gct      403
ggc gag tat ggt gcg gag gcc ctg gag ag  gtgaggctcc ctcccctgct        452
ccgacccggg ctcctcgccc gcccggaccc acaggccacc ctcaaccgtc ctggccccgg     512
acccaaaccc cacccctcac tctgcttctc cccgcag g atg ttc ctg tcc ttc        565
ccc acc acc aag acc tac ttc ccg cac ttc gac ctg agc cac ggc tct      613
gcc cag gtt aag ggc cac ggc aag aag gtg gcc gac gcg ctg acc aac      661
gcc gtg gcg cac gtg gac gac atg ccc aac gcg ctg tcc gcc ctg agc      709
gac ctg cac gcg cac aag ctt cgg gtg gac ccg gtc aac ttc aag          754
gtgagcggcg ggccgggagc gatctgggtc gaggggcgag atggcgcctt cctcgcaggg     814
cagaggatca cgcgggttgc gggaggtgta gcgcaggcgg cggctgcggg cctgggccct     874
cggccccact gaccctcttc tctgcacag ctc cta agc cac tgc ctg ctg gtg      927
acc ctg gcc gcc cac ctc ccc gcc gag ttc acc cct gcg gtg cac gcc      975
tcc ctg gac aag ttc ctg gct tct gtg agc acc gtg ctg acc tcc aaa     1023
tac cgt taagctggag cctcggtggc catgcttctt gccccttggg cctcccccca      1079
gcccctcctc cccttcctgc acccgtaccc ccgtggtctt tgaataaagt ctgagtgggc 1139
ggcagcctgt gtgtgcctga gttttttccc tcagcaaacg tgccaggcat gggcgtggac 1199
agcagctggg acacacatgg ctagaacctc tctgcagctg gatagggtag gaaaaggcag 1259
gggcgggagg aggggatgga ggagggaaag tggagccacc gcgaagtcca gctggaaaaa 1319
cgctggaccc tagagtgctt tgaggatgca tttgctcttt cccgagtttt attcccagac 1379
ttttcagatt caatgcaggt ttgctgaaat aatgaattta tccatcttta cgtttctggg 1439
cac 1442

<210> 115
<211> 142
<212> PRT
<213> Homo sapiens alpha1-globin

<400> 115

<210> 116
<211> 2206
<212> DNA
<213> Homo sapiens beta-globin

<220>
<221> CDS
<222> (351)..(442)

<220>
<221>  CDS
<222>  (573)..(795)

<220>
<221>  CDS
<222>  (1646)..(1771)

<400>  116
caatatgctt accaagctgt gattccaaat attacgtaaa tacacttgca aaggaggatg      60
tttttagtag caatttgtac tgatggtatg gggccaagag atatatctta gagggagggc     120
tgagggtttg aagtccaact cctaagccag tgccagaaga gccaaggaca ggtacggctg     180
tcatcactta gacctcaccc tgtggagcca caccctaggg ttggccaatc tactcccagg     240
agcagggagg gcaggagcca gggctgggca taaaagtcag ggcagagcca tctattgctt     300
acatttgctt ctgacacaac tgtgttcact agcaacctca aacagacacc atg gtg        356
cac ctg act cct gag gag aag tct gcc gtt act gcc ctg tgg ggc aag       404
gtg aac gtg gat gaa gtt ggt ggt gag gcc ctg ggc ag  gttggtatca        452
aggttacaag acaggtttaa ggagaccaat agaaactggg catgtggaga cagagaagac     512
tcttgggttt ctgataggca ctgactctct ctgcctattg gtctattttc ccacccttag     572
g ctg ctg gtg gtc tac cct tgg acc cag agg ttc ttt gag tcc ttt ggg     621
gat ctg tcc act cct gat gct gtt atg ggc aac cct aag gtg aag gct       669
cat ggc aag aaa gtg ctc ggt gcc ttt agt gat ggc ctg gct cac ctg       717
gac aac ctc aag ggc acc ttt gcc aca ctg agt gag ctg cac tgt gac       765
aag ctg cac gtg gat cct gag aac ttc agg gtgagtctat gggacgcttg         815
atgttttctt tccccttctt ttctatggtt aagttcatgt cataggaagg ggataagtaa     875
cagggtacag tttagaatgg gaaacagacg aatgattgca tcagtgtgga agtctcagga     935
tcgttttagt ttcttttatt tgctgttcat aacaattgtt ttcttttgtt taattcttgc     995
tttctttttt tttcttctcc gcaattttta ctattatact taatgcctta acattgtgta    1055
taacaaaagg aaatatctct gagatacatt aagtaactta aaaaaaaact ttacacagtc    1115
tgcctagtac attactattt ggaatatatg tgtgcttatt tgcatattca taatctccct    1175
actttatttt cttttatttt taattgatac ataatcatta tacatattta tgggttaaag    1235
tgtaatgttt taatatgtgt acacatattg accaaatcag ggtaattttg catttgtaat 1295
tttaaaaaat gctttcttct tttaatatac ttttttgttt atcttatttc taatactttc 1355
cctaatctct ttctttcagg gcaataatga tacaatgtat catgcctctt tgcaccattc 1415
taaagaataa cagtgataat ttctgggtta aggcaatagc aatatctctg catataaata 1475
tttctgcata taaattgtaa ctgatgtaag aggtttcata ttgctaatag cagctacaat 1535
ccagctacca ttctgctttt attttatggt tgggataagg ctggattatt ctgagtccaa 1595
gctaggccct tttgctaatc atgttcatac ctcttatctt cctcccacag ctc ctg 1651
ggc aac gtg ctg gtc tgt gtg ctg gcc cat cac ttt ggc aaa gaa ttc 1699
acc cca cca gtg cag gct gcc tat cag aaa gtg gtg gct ggt gtg gct 1747
aat gcc ctg gcc cac aag tat cac taagctcgct ttcttgctgt ccaatttcta 1801
ttaaaggttc ctttgttccc taagtccaac tactaaactg ggggatatta tgaagggcct 1861
tgagcatctg gattctgcct aataaaaaac atttattttc attgcaatga tgtatttaaa 1921
ttatttctga atattttact aaaaagggaa tgtgggaggt cagtgcattt aaaacataaa 1981
gaaatgaaga gctagttcaa accttgggaa aatacactat atcttaaact ccatgaaaga 2041
aggtgaggct gcaaacagct aatgcacatt ggcaacagcc cctgatgcat atgccttatt 2101
catccctcag aaaaggattc aagtagaggc ttgatttgga ggttaaagtt ttgctatgct 2161
gtattttaca ttacttattg ttttagctgt cctcatgaat gtctt 2206

<210> 117
<211> 147
<212> PRT
<213> Homo sapiens beta-globin

<400> 117